(19) Europäisches Patentamt
European Patent Office
Office européen des brevets

(11) EP 4 748 936 A1

(12) EUROPEAN PATENT APPLICATION
published in accordance with Art. 153(4) EPC

(43) Date of publication:
27.05.2026 Bulletin 2026/22

(21) Application number: 24843149.6

(22) Date of filing: 17.07.2024

(51) International Patent Classification (IPC):
C12N 15/53 (2006.01)   C12N 9/06 (2006.01)
C12N 11/02 (2006.01)   C12N 15/63 (2006.01)
C12Q 1/00 (2006.01)   C12Q 1/26 (2006.01)

(52) Cooperative Patent Classification (CPC):
C12N 9/0004; C12N 11/02; C12N 15/63;
C12Q 1/00; C12Q 1/26

(86) International application number:
PCT/JP2024/025602

(87) International publication number:
WO 2025/018360 (23.01.2025 Gazette 2025/04)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.07.2023 JP 2023117847

(71) Applicant: Provigate Inc.
Tokyo 113-0023 (JP)

(72) Inventors:
• SHINOHARA, Takeshi
Tokyo 113-0033 (JP)
• MINOURA, Itsushi
Tokyo 113-0033 (JP)
• HATTORI, Yuko
Tokyo 113-0033 (JP)
• MIYAZAWA, Yuya
Tokyo 113-0033 (JP)
• KAYASHIMA, Masatoshi
Tokyo 113-0033 (JP)

(74) Representative: Michalski Hüttermann & Partner
Patentanwälte mbB
Kaistraße 16A
40221 Düsseldorf (DE)

Remarks:
The complete document including Reference Table(s) and the Sequence Listing(s) can be downloaded from the EPO website

(54) **FRUCTOSYL-AMINO ACID OXIDASE, GENE, EXPRESSION VECTOR, GLYCATED PROTEIN SENSOR, GLYCATED PROTEIN MEASUREMENT METHOD, AND FRUCTOSYL-AMINO ACID OXIDASE PRODUCTION METHOD**

(57) [Object] For a glycated protein sensor including an immobilized enzyme, a technology for increasing measurement accuracy and the stability of the glycated protein sensor is essential. Unfortunately, there are no known enzymes that can increase the accuracy and stability of measurements that use a glycated protein sensor.

[Solution] The present invention provides a fructosyl amino acid oxidase including an amino acid sequence identical to an amino acid sequence set forth in SEQ ID NO: 1 except for an amino acid that is substituted, the amino acid being at a position corresponding to a position selected from the group consisting of positions 5, 58, 225, 277, and 440 of the amino acid sequence set forth in SEQ ID NO: 1, in a case where the amino acid sequence and the amino acid sequence set forth in SEQ ID NO: 1 are aligned with each other.

[FIG. 2]

# EP 4 748 936 A1

**Description**

TECHNICAL FIELD

The present invention relates to a fructosyl amino acid oxidase, to a gene encoding an amino acid sequence of a fructosyl amino acid oxidase, to an expression vector containing the gene, to a glycated protein sensor including a fructosyl amino acid oxidase immobilized on a support, to a method for measuring a glycated protein including detecting a glycated protein by using a reaction of a fructosyl amino acid oxidase immobilized on a support, and to a method for producing a fructosyl amino acid oxidase.

BACKGROUND ART

Measurements of glycated proteins are performed to diagnose diabetes or to provide a management index for glycemic control. For example, glycated hemoglobin and glycated albumin are frequently measured in clinical settings. Examples of known methods for measuring a glycated protein include electrophoresis, high-performance liquid chromatography, immunoassay, and enzymatic analysis.

A method for measuring a glycated protein by enzymatic analysis is a method in which, in a first step, a protein is degraded into amino acids or peptides by using a protease; in a second step, among the amino acids or peptides, an amino acid that has been glycated (hereinafter also referred to as a "glycated amino acid" or a "fructosyl amino acid") or a peptide containing a glycated amino acid (hereinafter also referred to as a "glycated peptide" or a "fructosyl peptide") is reacted with a fructosyl amino acid oxidase to generate hydrogen peroxide; and, in a third step, the hydrogen peroxide is converted into a color development reaction to measure an absorbance, or electrons emitted as a result of the decomposition of the hydrogen peroxide at an electrode are detected (see Patent Literature 1).

There are many known inventions characterized by an enzyme modified to improve measurement accuracy for a specific glycated amino acid or glycated peptide. Examples thereof include an amadoriase that is capable of reacting with various genotypes and enables highly accurate measurement of glycated hemoglobin in a sample containing glycated abnormal hemoglobin (see Patent Literature 2); an amadoriase obtained by substituting an amino acid of a specific amino acid sequence to improve a specific activity toward glycated substrates (see Patent Literature 3); an amadoriase obtained by substituting an amino acid of a specific amino acid sequence to enable activity to be retained in the presence of a surfactant (see Patent Literature 4 and 5); a fructosyl amino acid oxidase obtained by substituting an amino acid of a specific amino acid sequence to achieve high thermal stability (see Patent Literature 6 and 7); an amadoriase that is less likely to be influenced by oxygen concentration (see Patent Literature 8); and a method for measuring fructosyl lysine that uses a fructosyl amino acid oxidase carried by an Aspergillus oryzae RIB40 strain (see Patent Literature 9).

CITATION LIST

PATENT LITERATURE

PTL 1: International Publication No. 2019/221264
PTL 2: International Publication No. 2019/045052
PTL 3: International Publication No. 2016/159384
PTL 4: International Publication No. 2016/072520
PTL 5: International Publication No. 2015/020200
PTL 6: International Publication No. 2015/096621
PTL 7: Japanese Unexamined Patent Application Publication No. 2015-177790
PTL 8: International Publication No. 2016/063984
PTL 9: Japanese Unexamined Patent Application Publication No. 2009-000084

SUMMARY OF INVENTION

TECHNICAL PROBLEM

For a glycated protein sensor including an enzyme, a technology for increasing measurement accuracy and the stability of the sensor is essential. Unfortunately, there are no known enzymes that can increase the accuracy of measurements that use a glycated protein sensor and the stability of the sensor.

An object to be achieved by the present invention is to provide a fructosyl amino acid oxidase having physicochemical properties that contribute to the accuracy of measurements that use a glycated protein sensor and the stability of the sensor, and examples of such physicochemical properties include high enzymatic activity and high thermal stability.

SOLUTION TO PROBLEM

The present inventors discovered a fructosyl amino acid oxidase having novel physicochemical properties, among genes of unknown function. In addition, the present inventors discovered that substituting a specific amino acid of the fructosyl amino acid oxidase improves the physicochemical properties, and, accordingly, they arrived at the present invention.

Specifically, the present invention is a fructosyl amino acid oxidase having an amino acid sequence identical to an amino acid sequence set forth in SEQ ID NO: 1 except for an amino acid that is substituted, the amino acid being at a position corresponding to a position selected from the group consisting of positions 5, 58, 59, 98, 225, 277, 285, 355, and 440 of the amino acid sequence set forth in SEQ ID NO: 1, in a case where the amino acid sequence and the amino acid sequence set forth in SEQ ID NO: 1 are aligned with each other.

The present invention may be a fructosyl amino acid oxidase having a higher fructosyl amino acid oxidase activity and/or higher thermal stability than a fructosyl amino acid oxidase in which no amino acids are substituted, such as fructosyl amino acid oxidases consisting of the amino acid sequence set forth in SEQ ID NO: 1 or an amino acid sequence set forth in SEQ ID NO: 199. More specifically, the fructosyl amino acid oxidase is a fructosyl amino acid oxidase having a 4% or more higher fructosyl amino acid oxidase activity and/or 4% or more higher thermal stability than a fructosyl amino acid oxidase in which no amino acids are substituted, such as fructosyl amino acid oxidases consisting of the amino acid sequence set forth in SEQ ID NO: 1 or 199.

In another aspect, the present invention may be a fructosyl amino acid oxidase having a higher activity and/or higher thermal stability than a fructosyl amino acid oxidase in which no amino acids are substituted, such as fructosyl amino acid oxidases consisting of the amino acid sequence set forth in SEQ ID NO: 1 or 199, the activity and/or thermal stability being those exhibited while the fructosyl amino acid oxidase is immobilized on a support.

In another aspect, the present invention may be a fructosyl amino acid oxidase having an activity exhibited after heat treatment of the fructosyl amino acid oxidase, and the activity is higher than an activity of a fructosyl amino acid oxidase in which no amino acids are substituted, such as fructosyl amino acid oxidases consisting of the amino acid sequence set forth in SEQ ID NO: 1 or 199, the activity being an activity exhibited after the heat treatment of the fructosyl amino acid oxidase in which no amino acids are substituted. Conditions for the heat treatment include, for example, a temperature of 45°C or 48°C and a duration of 15 minutes.

In another aspect, the present invention may be a fructosyl amino acid oxidase having a higher thermal stability for a heat treatment than a fructosyl amino acid oxidase in which no amino acids are substituted, such as fructosyl amino acid oxidases consisting of the amino acid sequence set forth in SEQ ID NO: 1 or 199. Conditions for the heat treatment include, for example, a temperature of 45°C or 48°C and a duration of 15 minutes.

In another aspect, the present invention may be a fructosyl amino acid oxidase having a specific reactivity with fructosyl lysine. Examples thereof include a fructosyl amino acid oxidase having reactivities with fructosyl valine, a fructosyl valine-containing peptide, and fructosyl glycine, each of the reactivities being 20 or less based on the reactivity with fructosyl lysine, which is taken as 100.

In another aspect, the present invention may be a fructosyl amino acid oxidase having an amino acid sequence that shows a homology of 75% or greater to the amino acid sequence set forth in SEQ ID NO: 1. The amino acid sequence of the fructosyl amino acid oxidase of the present invention may be an amino acid sequence that satisfies at least one of (1) to (9) below:

(1) the amino acid at a position corresponding to position 5 of the amino acid sequence set forth in SEQ ID NO: 1 is one selected from the group consisting of alanine, arginine, aspartic acid, cysteine, glutamine, glutamic acid, glycine, histidine, isoleucine, leucine, lysine, methionine, phenylalanine, proline, serine, threonine, tryptophan, tyrosine, and valine;

(2) the amino acid at a position corresponding to position 58 of the amino acid sequence set forth in SEQ ID NO: 1 is one selected from the group consisting of alanine, arginine, asparagine, aspartic acid, cysteine, glutamine, glutamic acid, glycine, histidine, isoleucine, leucine, lysine, phenylalanine, proline, serine, threonine, tryptophan, tyrosine, and valine;

(3) the amino acid at a position corresponding to position 59 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;

(4) the amino acid at a position corresponding to position 98 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;

(5) the amino acid at a position corresponding to position 225 of the amino acid sequence set forth in SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine;

(6) the amino acid at a position corresponding to position 277 of the amino acid sequence set forth in SEQ ID NO: 1 is one selected from the group consisting of alanine, arginine, asparagine, aspartic acid, cysteine, glutamine, glutamic acid, glycine, histidine, isoleucine, leucine, methionine, phenylalanine, proline, serine, threonine, tryptophan, tyrosine, and valine;

(7) the amino acid at a position corresponding to position 285 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;

(8) the amino acid at a position corresponding to position 355 of the amino acid sequence set forth in SEQ ID NO: 1 is one selected from the group consisting of alanine, arginine, asparagine, cysteine, glutamine, glutamic acid, glycine, histidine, isoleucine, leucine, lysine, methionine, phenylalanine, proline, serine, threonine, tryptophan, tyrosine, and valine; and

(9) the amino acid at a position corresponding to position 440 of the amino acid sequence set forth in SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine.

In another aspect, the present invention may be a fructosyl amino acid oxidase having an amino acid sequence that shows a homology of 79% or greater to an amino acid sequence set forth in SEQ ID NO: 1 and in which an amino acid is histidine, serine, threonine, glycine, alanine, valine, leucine, isoleucine, or phenylalanine or is phenylalanine, alanine, or isoleucine, the amino acid being at a position corresponding to position 58 of the amino acid sequence set forth in SEQ ID NO: 1 in a case where the amino acid sequence and the amino acid sequence set forth in SEQ ID NO: 1 are aligned with each other.

In another aspect, the present invention is a fructosyl amino acid oxidase having an amino acid sequence set forth in one of SEQ ID NOs: 2 to 198 and 200 to 218. In another aspect, the present invention may be a fructosyl amino acid oxidase having an amino acid sequence that is identical to the above-mentioned amino acid sequence except that one, two, three, four, five, six, seven, eight, nine, or ten amino acids are deleted, substituted, added, and/or inserted. Furthermore, the present invention provides a gene encoding the amino acid sequence of any of the above-described fructosyl amino acid oxidases and also provides an expression vector containing the gene.

Furthermore, the present invention provides a glycated protein sensor including a fructosyl amino acid oxidase and a support. In the glycated protein sensor, the fructosyl amino acid oxidase may be immobilized on the support as a result of crosslinking induced by an amine-reactive crosslinking agent.

In the sensor of the present invention, an output value of the sensor, which is a value after the sensor is allowed to stand at a temperature of 65°C or 80°C for 20 minutes, may be equal to or greater than 60% of an output value of the sensor, which is a value before the sensor is allowed to stand. Furthermore, an output value of the sensor, which is a value after the sensor is allowed to stand at a temperature of 75°C and a relative humidity of 60% for one day or two days, may be equal to or greater than 40%, 50%, 60%, or 70% of an output value of the sensor, which is a value before the sensor is allowed to stand.

Furthermore, the present invention provides a method for measuring a glycated protein including detecting a glycated protein by using a reaction of a fructosyl amino acid oxidase and also provides a method for producing a fructosyl amino acid oxidase including an amino acid substitution step of substituting an amino acid of an amino acid sequence of the fructosyl amino acid oxidase, the amino acid being at a position corresponding to a position selected from the group consisting of positions 5, 58, 225, 277, and 440 of an amino acid sequence set forth in SEQ ID NO: 1, in a case where the amino acid sequence and the amino acid sequence set forth in SEQ ID NO: 1 are aligned with each other. Regarding the fructosyl amino acid oxidase used in the production method of the present invention, its amino acid sequence before amino acid substitution may be an amino acid sequence that shows a high homology to the amino acid sequence set forth in SEQ ID NO: 1, and the homology may be, for example, 50% or greater, 60% or greater, 70% or greater, 75% or greater, or 79% or greater.

ADVANTAGEOUS EFFECTS OF INVENTION

Fructosyl amino acid oxidases of the present invention are stable to heat while they are immobilized on a support and/or are not immobilized on a support. In particular, the fact that the fructosyl amino acid oxidases of the present invention are stable to heat while they are immobilized on a support is advantageous in terms of improving the long-term stability of a glycated protein sensor including an immobilized enzyme. Such a glycated protein sensor can provide highly reliable measurement results over a long period of time because, during storage of the sensor, a reduction in the enzymatic activity is inhibited.

Furthermore, the present invention can provide a fructosyl amino acid oxidase having higher enzymatic activity and thermal stability than wild-type fructosyl amino acid oxidases. A production method of the present invention can produce a variant having high activity and/or thermal stability, from a known fructosyl amino acid oxidase.

BRIEF DESCRIPTION OF DRAWINGS

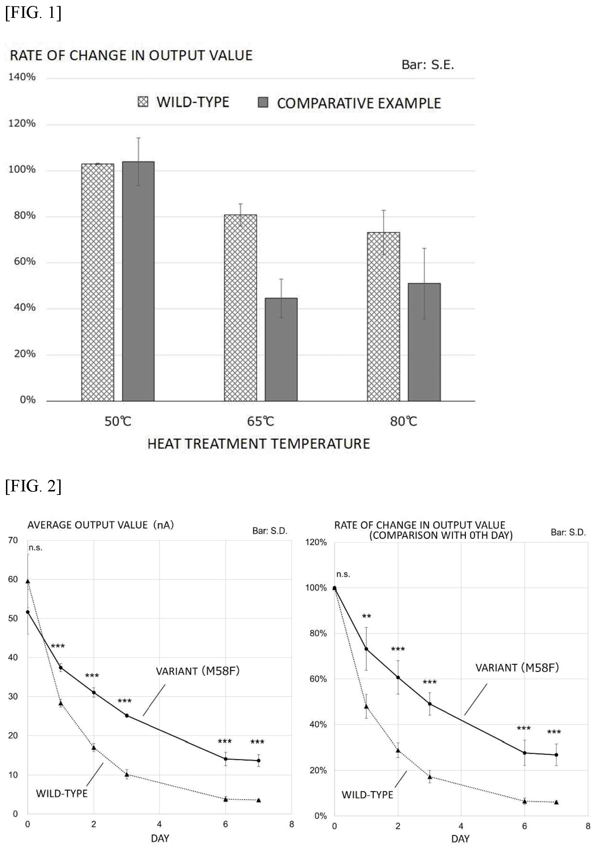

Fig. 1 presents a graph showing the results of an experiment in which a thermal stability of a sensor including a fructosyl amino acid oxidase having an amino acid sequence set forth in SEQ ID NO: 1 was evaluated. Fig. 2 presents graphs showing an output value (left) and a rate of change in the output value (right) of sensors, one of which includes a fructosyl amino acid oxidase (wild-type) having the amino acid sequence set forth in SEQ ID NO: 1, and the other of which includes a fructosyl amino acid oxidase (variant M58F) having an amino acid sequence set forth in SEQ ID NO: 38. A statistically significant difference between the two groups was calculated using a t-test. In the figure, "***" indicates that a p-value was less than 0.001, "**" indicates that the p-value was less than 0.01, and "n.s." indicates that no statistically significant difference was observed.

DESCRIPTION OF EMBODIMENTS

Fructosyl amino acid oxidases of the present invention have high enzymatic activity and high thermal stability. Furthermore, a fructosyl amino acid oxidase of an embodiment of the present invention has high thermal stability while it is immobilized on a support. Furthermore, the present invention provides a gene encoding the amino acid sequence of a fructosyl amino acid oxidase and also provides an expression vector containing the gene. In addition, the present invention provides a glycated protein sensor including a support and a fructosyl amino acid oxidase immobilized on the support, provides a method for measuring a glycated protein including detecting a glycated protein by using a reaction of a fructosyl amino acid oxidase immobilized on a support, and provides a method for producing a fructosyl amino acid oxidase. The present invention will be described in detail below with reference to embodiments.

A fructosyl amino acid oxidase is an enzyme that acts on an amino acid containing a glycated $\alpha$-amino group and/or a glycated $\varepsilon$-amino group or on a peptide containing such an amino acid and which produces hydrogen peroxide in the process of deglycating a glycated amino acid. A fructosyl amino acid oxidase (hereinafter also referred to as an "FAOD") is also referred to as an "amadoriase", a "ketoamine oxidase", or a "fructosyl amine oxidase". Fructosyl amino acid oxidases include a fructosyl peptide oxidase (hereinafter also referred to as an "FPOD" or an "FPOX"), which acts on glycated peptides.

Substrates of fructosyl amino acid oxidases are glycated amino acids and/or glycated peptides. Specific examples of the glycated amino acids include $\varepsilon$-fructosyl lysine (also referred to as "fructosyl lysine"), $\alpha$-fructosyl valine (also referred to as "fructosyl valine"), $\alpha$-fructosyl glycine (also referred to as "fructosyl glycine"), $\alpha$-fructosyl histiditine (also referred to as "fructosyl histidine"), $\alpha$-fructosyl leucine (also referred to as "fructosyl leucine"), and $\alpha$-fructosyl serine (also referred to as "fructosyl serine").

Specific examples of the glycated peptides include peptides composed of 2 to 10 amino acids, with one or more of the amino acids being glycated amino acids; the number of amino acids is preferably 2 to 6 and more preferably 2 or 3. One example is $\alpha$-fructosyl valyl histidine (also referred to as "fructosyl valyl histidine").

Fructosyl amino acid oxidases of the present invention have the following physicochemical properties. The fructosyl amino acid oxidases have an optimum pH range of pH 7 to 9, an operable pH range of pH 5 to 9, and an operable temperature range of 20 to 80°C and are soluble in a buffer solution. Specific examples of the buffer solution include Tris-hydrochloride buffers and phosphate buffered salines (PBS).

A fructosyl amino acid oxidase of the present invention may specifically react with a particular glycated amino acid or a peptide containing the amino acid. In an embodiment, a fructosyl amino acid oxidase is highly specific for fructosyl lysine. The expression "being highly specific for fructosyl lysine" means that a reactivity with a different glycated amino acid or a peptide containing a different glycated amino acid is 20 or less based on the reactivity with fructosyl lysine or a peptide containing fructosyl lysine, which is taken as 100; the reactivity with a different glycated amino acid or a peptide containing a different glycated amino acid is preferably 10 or less, more preferably 5 or less, and even more preferably 1 or less. The different glycated amino acid is, for example, one or more selected from fructosyl valine, fructosyl glycine, fructosyl histidine, fructosyl leucine, and fructosyl serine.

Fructosyl amino acid oxidases can be classified into three groups according to their substrate specificity. Fructosyl amino acid oxidases belonging to the first group are highly specific for amino acids containing a glycated α-amino group and/or for peptides containing such an amino acid. Fructosyl amino acid oxidases belonging to the second group are highly specific for amino acids containing a glycated ε-amino group and/or for peptides containing such an amino acid. Fructosyl amino acid oxidases belonging to the third group are highly specific for amino acids containing a glycated α-amino group, for peptides containing such an amino acid, for amino acids containing a glycated ε-amino group, and/or for peptides containing such an amino acid. The fructosyl amino acid oxidases of the present invention may be fructosyl amino acid oxidases belonging to any of these groups.

In another embodiment, a fructosyl amino acid oxidase is highly specific for fructosyl valyl histidine. The expression "being highly specific for fructosyl valyl histidine" means that a reactivity with a different glycated amino acid or a different glycated peptide is 20 or less based on the reactivity with fructosyl valyl histidine, which is taken as 100; the reactivity with a different glycated amino acid or a different fructosy peptide is preferably 10 or less, more preferably 5 or less, and even more preferably 1 or less. The different glycated amino acid is, for example, one or more selected from fructosyl valine, fructosyl lysine, fructosyl glycine, fructosyl histidine, fructosyl leucine, and fructosyl serine, and the different fructosyl peptide is a peptide that contains a glycated amino acid and is different from fructosyl valyl histidine.

A fructosyl amino acid oxidase of the present invention may specifically react with a particular glycated amino acid or a peptide containing the amino acid. In an embodiment, a fructosyl amino acid oxidase is highly specific for fructosyl valine. The expression "being highly specific for fructosyl valine" means that a reactivity with a different glycated amino acid or a peptide containing a different glycated amino acid is 20 or less based on the reactivity with fructosyl valine or a peptide containing fructosyl valine, which is taken as 100; the reactivity with a different glycated amino acid or a peptide containing a different glycated amino acid is preferably 10 or less, more preferably 5 or less, and even more preferably 1 or less. The different glycated amino acid is, for example, one or more selected from fructosyl lysine, fructosyl glycine, fructosyl histidine, fructosyl leucine, and fructosyl serine.

The inventors discovered novel fructosyl amino acid oxidases among genes of unknown function. Among them, there was a fructosyl amino acid oxidase having a significantly high thermal stability exhibited while it was immobilized on a support and/or was not immobilized on a support. Accordingly, based on the amino acid sequence of the fructosyl amino acid oxidase, the inventors created variants of the fructosyl amino acid oxidase.

The amino acid sequence set forth in SEQ ID NO: 1 is the amino acid sequence of a fructosyl amino acid oxidase derived from Aspergillus pseudotamarii, which belongs to the genus Aspergillus. So far, the protein has been assumed to be a type of FAD-dependent oxidoreductase (Accession Number XP_031920077), based on a homology of the sequence, but it has not been known that the protein including the amino acid sequence set forth in SEQ ID NO: 1 could function as a fructosyl amino acid oxidase. The present inventors discovered that the fructosyl amino acid oxidase including the amino acid sequence set forth in SEQ ID NO: 1 exhibits a significantly high thermal stability while it is immobilized on a support and that the fructosyl amino acid oxidase has a very high substrate specificity for a particular glycated amino acid and/or glycated peptide.

The present invention provides a fructosyl amino acid oxidase including an amino acid sequence that has a high homology to SEQ ID NO: 1 and in which one or more amino acids are substituted, the amino acids being at positions corresponding to positions selected from the group consisting of positions 5, 58, 225, 277, and 440 of the amino acid sequence set forth in SEQ ID NO: 1, in a case where the amino acid sequence and the amino acid sequence set forth in SEQ ID NO: 1 are aligned with each other. Regarding fructosyl amino acid oxidases consisting of a known amino acid sequence, even if they satisfy the above-mentioned conditions, they are excluded from the fructosyl amino acid oxidases of the present invention.

The amino acid sequence that has a high homology to SEQ ID NO: 1 may include an amino acid sequence having a homology, to the amino acid sequence of SEQ ID NO: 1, of 30% or greater, 35% or greater, 40% or greater, 45% or greater, 50% or greater, 51% or greater, 52% or greater, 53% or greater, 54% or greater, 55% or greater, 56% or greater, 57% or greater, 58% or greater, 59% or greater, 60% or greater, 61% or greater, 62% or greater, 63% or greater, 64% or greater, 65% or greater, 66% or greater, 67% or greater, 68% or greater, 69% or greater, 70% or greater, 71% or greater, 72% or greater, 73% or greater, 74% or greater, 75% or greater, 76% or greater, 77% or greater, 78% or greater, 79% or greater, 80% or greater, 81% or greater, 82% or greater, 83% or greater, 84% or greater, 85% or greater, 86% or greater, 87% or greater, 88% or greater, 89% or greater, 90% or greater, 91% or greater, 92% or greater, 93% or greater, 94% or greater, 95% or greater, 96% or greater, 97% or greater, 98% or greater, or 99% or greater. These homology values are values rounded to the nearest whole number (the same applies hereinafter). The homology of amino acid sequences can be calculated, for example, by using default parameters in BLAST of the National Center for Biotechnology Information (NCBI) (http://www.ncbi.nlm.nih.gov/BLAST/).

In the amino acid sequence set forth in SEQ ID NO: 1, the amino acid at position 5 is an asparagine. In an embodiment of the present invention, a fructosyl amino acid oxidase has an amino acid sequence in which an amino acid at a position corresponding to position 5 of SEQ ID NO: 1 is one selected from the group consisting of alanine, arginine, aspartic acid, cysteine, glutamine, glutamic acid, glycine, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

A fructosyl amino acid oxidase set forth in SEQ ID NO: 199 is one in which the amino acid at a position corresponding to position 5 of SEQ ID NO: 1 is an isoleucine in a case where the amino acid sequence and the amino acid sequence set forth in SEQ ID NO: 1 are aligned with each other; however, the amino acid sequence set forth in SEQ ID NO: 199 is excluded from the amino acid sequences of the fructosyl amino acid oxidases of the invention of the present application. The amino acid sequence of a fructosyl amino acid oxidase described in GenBank accession number BAD54824 and the amino acid sequence of a fructosyl amino acid oxidase disclosed in Patent Literature 9 are those in which the amino acid at a position corresponding to position 5 of SEQ ID NO: 1 is a lysine in a case where the amino acid sequences and the amino acid sequence set forth in SEQ ID NO: 1 are aligned with each other; however, the amino acid sequences of these known fructosyl amino acid oxidases are excluded from the amino acid sequences of the fructosyl amino acid oxidases of the invention of the present application.

In the amino acid sequence set forth in SEQ ID NO: 1, the amino acid at position 58 is a methionine. In an embodiment of the present invention, a fructosyl amino acid oxidase has an amino acid sequence in which an amino acid at a position corresponding to position 58 of SEQ ID NO: 1 is one selected from the group consisting of alanine, arginine, asparagine, aspartic acid, cysteine, glutamine, glutamic acid, glycine, histidine, isoleucine, leucine, lysine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 59 is a glutamic acid. In an embodiment of the present invention, a fructosyl amino acid oxidase has an amino acid sequence in which an amino acid at a position corresponding to position 59 of SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 98 is a glutamic acid. In an embodiment of the present invention, a fructosyl amino acid oxidase has an amino acid sequence in which an amino acid at a position corresponding to position 98 of SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 225 is a glycine. In an embodiment of the present invention, a fructosyl amino acid oxidase has an amino acid sequence in which an amino acid at a position corresponding to position 225 of SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 277 is a lysine. In an embodiment of the present invention, a fructosyl amino acid oxidase has an amino acid sequence in which an amino acid at a position corresponding to position 277 of SEQ ID NO: 1 is one selected from the group consisting of alanine, arginine, asparagine, aspartic acid, cysteine, glutamine, glutamic acid, glycine, histidine, isoleucine, leucine, methionine, phenylalanine, proline, serine, threonine, tryptophan, tyrosine, and valine, in a case where the amino acid sequence and the amino acid sequence set forth in SEQ ID NO: 1 are aligned with each other.

Regarding the fructosyl amino acid oxidase disclosed in Patent Literature 9, its amino acid sequence is one in which the amino acid at a position corresponding to position 277 of SEQ ID NO: 1 is an asparagine in a case where the amino acid sequence and the amino acid sequence set forth in SEQ ID NO: 1 are aligned with each other; however, the amino acid sequence of the fructosyl amino acid oxidase disclosed in Patent Literature 9 is excluded from the amino acid sequences of the fructosyl amino acid oxidases of the invention of the present application.

In the amino acid sequence set forth in SEQ ID NO: 1, the amino acid at position 285 is a glutamic acid. In an embodiment of the present invention, a fructosyl amino acid oxidase has an amino acid sequence in which an amino acid at a position corresponding to position 285 of SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 355 is an aspartic acid. In an embodiment of the present invention, a fructosyl amino acid oxidase has an amino acid sequence in which an amino acid at a position corresponding to position 355 of SEQ ID NO: 1 is one selected from the group consisting of alanine, arginine, asparagine, cysteine, glutamine, glutamic acid, glycine, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

In the amino acid sequence set forth in SEQ ID NO: 1, the amino acid at position 440 is a glycine. In an embodiment of the present invention, a fructosyl amino acid oxidase has an amino acid sequence in which an amino acid at a position corresponding to position 440 of SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine, in a case where the amino acid sequence and the amino acid sequence set forth in SEQ ID NO: 1 are aligned with each other.

Furthermore, another embodiment of the present invention is a fructosyl amino acid oxidase having an amino acid sequence in which the above-mentioned substituted amino acid is a plurality of amino acids. Specific examples include fructosyl amino acid oxidases having an amino acid sequence in which amino acids are substituted, the amino acids being at positions corresponding to two, three, four, five, six, seven, eight, or nine positions selected from the group consisting of positions 5, 58, 59, 98, 225, 277, 285, 355, and 440.

Examples of the amino acid sequences of fructosyl amino acid oxidases of the present invention are shown below. An embodiment of the present invention may be a fructosyl amino acid oxidase including or consisting of one of the amino acid sequences shown below. Another embodiment may be a fructosyl amino acid oxidase consisting of one of the amino acid sequences shown below.

## [Table 1]

Amino Acid Sequence Identical to Amino Acid Sequence Set Forth in SEQ ID NO: 1 Except for Substitution of N5

| SEQ ID NO: | Variation | Amino Acid Sequence |
|---|---|---|
| 2 | N5D | MASSDLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 3 | N5E | MASSELTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 4 | N5K | MASSKLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 5 | N5R | MASSRLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 6 | N5H | MASSHLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 7 | N5Q | MASSQLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 8 | N5S | MASSSLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 9 | N5T | MASSTLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 10 | N5Y | MASSYLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 11 | N5C | MASSCLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 12 | N5G | MASSGLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 13 | N5A | MASSALTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 14 | N5V | MASSVLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 15 | N5P | MASSPLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 16 | N5L | MASSLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 17 | N5I | MASSILTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 18 | N5F | MASSFLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 19 | N5M | MASSMLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 20 | N5W | MASSWLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

[Table 2]

Amino Acid Sequence Identical to Amino Acid Sequence Set Forth in SEQ ID NO: 1 Except for Substitution of M58 with Another Amino Acid

| SEQ ID NO: | Variation | Amino Acid Sequence |
|---|---|---|
| 21 | M58D | MASSNLTLTSSILIVGAGTWGCSTALHLARRGYKNVTVLDPHPVPSPIAAGNDINKIDEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 22 | M58E | MASSNLTLTSSILIVGAGTWGCSTALHLARRGYKNVTVLDPHPVPSPIAAGNDINKIEEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 23 | M58K | MASSNLTLTSSILIVGAGTWGCSTALHLARRGYKNVTVLDPHPVPSPIAAGNDINKIKEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 24 | M58R | MASSNLTLTSSILIVGAGTWGCSTALHLARRGYKNVTVLDPHPVPSPIAAGNDINKIREHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 25 | M58H | MASSNLTLTSSILIVGAGTWGCSTALHLARRGYKNVTVLDPHPVPSPIAAGNDINKIHEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 26 | M58N | MASSNLTLTSSILIVGAGTWGCSTALHLARRGYKNVTVLDPHPVPSPIAAGNDINKINEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 27 | M58Q | MASSNLTLTSSILIVGAGTWGCSTALHLARRGYKNVTVLDPHPVPSPIAAGNDINKIQEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 28 | M58S | MASSNLTLTSSILIVGAGTWGCSTALHLARRGYKNVTVLDPHPVPSPIAAGNDINKISEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 29 | M58T | MASSNLTLTSSILIVGAGTWGCSTALHLARRGYKNVTVLDPHPVPSPIAAGNDINKITEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 30 | M58Y | MASSNLTLTSSILIVGAGTWGCSTALHLARRGYKNVTVLDPHPVPSPIAAGNDINKIYEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 31 | M58C | MASSNLTLTSSILIVGAGTWGCSTALHLARRGYKNVTVLDPHPVPSPIAAGNDINKICEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 32 | M58G | MASSNLTLTSSILIVGAGTWGCSTALHLARRGYKNVTVLDPHPVPSPIAAGNDINKIGEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 33 | M58A | MASSNLTLTSSILIVGAGTWGCSTALHLARRGYKNVTVLDPHPVPSPIAAGNDINKIAEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 34 | M58V | MASSNLTLTSSILIVGAGTWGCSTALHLARRGYKNVTVLDPHPVPSPIAAGNDINKIVEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 35 | M58P | MASSNLTLTSSILIVGAGTWGCSTALHLARRGYKNVTVLDPHPVPSPIAAGNDINKIPEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 36 | M58L | MASSNLTLTSSILIVGAGTWGCSTALHLARRGYKNVTVLDPHPVPSPIAAGNDINKILEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 37 | M58I | MASSNLTLTSSILIVGAGTWGCSTALHLARRGYKNVTVLDPHPVPSPIAAGNDINKIIEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 38 | M58F | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 39 | M58W | MASSNLTLTSSILIVGAGTWGCSTALHLARRGYKNVTVLDPHPVPSPIAAGNDINKIWEHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

[Table 3]

| Amino Acid Sequence Identical to Amino Acid Sequence Set Forth in SEQ ID NO: 1 Except for Substitution of G225 with Another Amino Acid | | |
| SEQ ID NO: | Variation | Amino Acid Sequence |
| --- | --- | --- |
| 40 | G225D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGADSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 41 | G225E | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAESDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 42 | G225K | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAKSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 43 | G225R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGARSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 44 | G225H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAHSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 45 | G225N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 46 | G225Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAQSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 47 | G225S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGASSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 48 | G225T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGATSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 49 | G225Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAYSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 50 | G225C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGACSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 51 | G225A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 52 | G225V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAVSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 53 | G225P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAPSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 54 | G225L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGALSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 55 | G225I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAISDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 56 | G225F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAFSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 57 | G225M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAMSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 58 | G225W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAWSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

[Table 4]

| | | Amino Acid Sequence Identical to Amino Acid Sequence Set Forth in SEQ ID NO: 1 Except for Substitution of G440 with Another Amino Acid |
|---|---|---|
| **SEQ ID NO:** | **Variation** | **Amino Acid Sequence** |
| 59 | G440D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIDDDKCRL |
| 60 | G440E | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIEDDKCRL |
| 61 | G440K | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIKDDKCRL |
| 62 | G440R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIRDDKCRL |
| 63 | G440H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIHDDKCRL |
| 64 | G440N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKINDDKCRL |
| 65 | G440Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIQDDKCRL |
| 66 | G440S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKISDDKCRL |
| 67 | G440T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKITDDKCRL |
| 68 | G440Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIYDDKCRL |
| 69 | G440C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKICDDKCRL |
| 70 | G440A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |
| 71 | G440V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIVDDKCRL |
| 72 | G440P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIPDDKCRL |
| 73 | G440L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKILDDKCRL |
| 74 | G440I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIIDDKCRL |
| 75 | G440F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIFDDKCRL |
| 76 | G440M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 77 | G440W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIWDDKCRL |

[Table 5]

| Amino Acid Sequence Identical to Amino Acid Sequence Set Forth in SEQ ID NO: 1 Except for Substitution of E59 with Another Amino Acid | | |
|---|---|---|
| SEQ ID NO: | Variation | Amino Acid Sequence |
| 78 | E59D | MASSNLTLTSSILIVGAGTWGCSTALHLARRGYKNVTVLDPHPVPSPIAAGNDINKIMDHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 79 | E59K | MASSNLTLTSSILIVGAGTWGCSTALHLARRGYKNVTVLDPHPVPSPIAAGNDINKIMKHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 80 | E59R | MASSNLTLTSSILIVGAGTWGCSTALHLARRGYKNVTVLDPHPVPSPIAAGNDINKIMRHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 81 | E59H | MASSNLTLTSSILIVGAGTWGCSTALHLARRGYKNVTVLDPHPVPSPIAAGNDINKIMHHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 82 | E59N | MASSNLTLTSSILIVGAGTWGCSTALHLARRGYKNVTVLDPHPVPSPIAAGNDINKIMNHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 83 | E59Q | MASSNLTLTSSILIVGAGTWGCSTALHLARRGYKNVTVLDPHPVPSPIAAGNDINKIMQHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 84 | E59S | MASSNLTLTSSILIVGAGTWGCSTALHLARRGYKNVTVLDPHPVPSPIAAGNDINKIMSHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 85 | E59T | MASSNLTLTSSILIVGAGTWGCSTALHLARRGYKNVTVLDPHPVPSPIAAGNDINKIMTHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 86 | E59Y | MASSNLTLTSSILIVGAGTWGCSTALHLARRGYKNVTVLDPHPVPSPIAAGNDINKIMYHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 87 | E59C | MASSNLTLTSSILIVGAGTWGCSTALHLARRGYKNVTVLDPHPVPSPIAAGNDINKIMCHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 88 | E59G | MASSNLTLTSSILIVGAGTWGCSTALHLARRGYKNVTVLDPHPVPSPIAAGNDINKIMGHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 89 | E59A | MASSNLTLTSSILIVGAGTWGCSTALHLARRGYKNVTVLDPHPVPSPIAAGNDINKIMAHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 90 | E59V | MASSNLTLTSSILIVGAGTWGCSTALHLARRGYKNVTVLDPHPVPSPIAAGNDINKIMVHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 91 | E59P | MASSNLTLTSSILIVGAGTWGCSTALHLARRGYKNVTVLDPHPVPSPIAAGNDINKIMPHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 92 | E59L | MASSNLTLTSSILIVGAGTWGCSTALHLARRGYKNVTVLDPHPVPSPIAAGNDINKIMLHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 93 | E59I | MASSNLTLTSSILIVGAGTWGCSTALHLARRGYKNVTVLDPHPVPSPIAAGNDINKIMIHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 94 | E59F | MASSNLTLTSSILIVGAGTWGCSTALHLARRGYKNVTVLDPHPVPSPIAAGNDINKIMFHREVKASETDPWSIAFSTCTRAALKGWKTDPVFQPYFHETGAIVSGHTPSLIKHIEEHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLLDFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDADTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

(continued)

| Amino Acid Sequence Identical to Amino Acid Sequence Set Forth in SEQ ID NO: 1 Except for Substitution of E59 with Another Amino Acid | | |
|---|---|---|
| SEQ ID NO: | Variation | Amino Acid Sequence |
| 95 | E59M | MASSNLTLTSSILIVGAGTWGCSTALHLARRGYKNVTVLDPHPVPSPIAAGNDINKIMMHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 96 | E59W | MASSNLTLTSSILIVGAGTWGCSTALHLARRGYKNVTVLDPHPVPSPIAAGNDINKIMWHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |

[Table 6]

| Amino Acid Sequence Identical to Amino Acid Sequence Set Forth in SEQ ID NO: 1 Except for Substitution of E98 with Another Amino Acid | | |
|---|---|---|
| SEQ ID NO: | Variation | Amino Acid Sequence |
| 97 | E98D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHDTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 98 | E98K | MASSNLTLTSSILIVGAGTWGCSTALHLARRGYKNVTVLDPHPVPSPIAAGNDINKIMEHREVKASETDPWSIAFSTCTRAALKGWKTDPVFQPYFHKTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 99 | E98R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHRTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 100 | E98H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHHTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 101 | E98N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHNTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 102 | E98Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHQTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 103 | E98S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHSTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 104 | E98T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHTTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 105 | E98Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHYTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 106 | E98C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHCTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |
| 107 | E98G | MASSNLTLTSSILIVGAGTWGCSTALHLARRGYKNVTVLDPHPVPSPIAAGNDINKIMEHREVKASETDPWSIAFSTCTRAALKGWKTDPVFQPYFHCTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , MASSNLTLTSSILIVGAGTWGCSTALHLARRGYKNVTVLDPHPVPSPIAAGNDINKIMEHREVKASETDPWSIAFSTCTRAALKGWKTDPVFQPYFHGTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL , |

(continued)

| Amino Acid Sequence Identical to Amino Acid Sequence Set Forth in SEQ ID NO: 1 Except for Substitution of E98 with Another Amino Acid | | |
|---|---|---|
| SEQ ID NO: | Variation | Amino Acid Sequence |
| 108 | E98A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHATGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 109 | E98V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHVTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 110 | E98P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHPTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 111 | E98L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHLTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 112 | E98I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHITGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 113 | E98F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHFTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 114 | E98M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHMTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 115 | E98W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHWTGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

## [Table 7]

| SEQ ID NO: | Variation | Amino Acid Sequence Identical to Amino Acid Sequence Set Forth in SEQ ID NO: 1 Except for Substitution of K277 with Another Amino Acid |
|---|---|---|
| 116 | K277D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDDHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 117 | K277E | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDEHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 118 | K277R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDRHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 119 | K277H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDHHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 120 | K277N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDNHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 121 | K277Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDQHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 122 | K277S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDSHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 123 | K277T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDTHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 124 | K277Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDYHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 125 | K277C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDCHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 126 | K277G | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDGHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 127 | K277A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDAHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 128 | K277V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDVHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 129 | K277P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDPHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 130 | K277L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDLHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 131 | K277I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDIHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 132 | K277F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDFHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 133 | K277M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDMHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 134 | K277W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDWHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

## [Table 8]

Amino Acid Sequence Identical to Amino Acid Sequence Set Forth in SEQ ID NO: 1 Except for Substitution of E285 with Another Amino Acid

| SEQ ID NO: | Variation | Amino Acid Sequence |
|---|---|---|
| 135 | E285D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDDHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 136 | E285K | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDKHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 137 | E285R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDRHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 138 | E285H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDHHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 139 | E285N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDNHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 140 | E285Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDQHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 141 | E285S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDSHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 142 | E285T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDTHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 143 | E285Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDYHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 144 | E285C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDCHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 145 | E285G | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDGHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 146 | E285A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDAHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 147 | E285V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDVHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 148 | E285P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDPHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 149 | E285L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDLHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 150 | E285I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDIHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 151 | E285F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDFHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 152 | E285M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDMHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 153 | E285W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDWHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

[Table 9]

| SEQ ID NO: | Variation | Amino Acid Sequence |
|---|---|---|
| | Amino Acid Sequence Identical to Amino Acid Sequence Set Forth in SEQ ID NO: 1 Except for Substitution of D355 with Another Amino Acid | |
| 154 | D355E | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIERHPEYRSLLLAVGGSGNGAMQMSTIGGFITDALEGHLQKELKHALRWRPEIATERDWKDTQNRFGGPNKVMDFQKVGENEWTKIGDDKCRL |
| 155 | D355K | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIKRHPEYRSLLLAVGGSGNGAMQMSTIGGFITDALEGHLQKELKHALRWRPEIATERDWKDTQNRFGGPNKVMDFQKVGENEWTKIGDDKCRL |
| 156 | D355R | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIRRHPEYRSLLLAVGGSGNGAMQMSTIGGFITDALEGHLQKELKHALRWRPEIATERDWKDTQNRFGGPNKVMDFQKVGENEWTKIGDDKCRL |
| 157 | D355H | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIHRHPEYRSLLLAVGGSGNGAMQMSTIGGFITDALEGHLQKELKHALRWRPEIATERDWKDTQNRFGGPNKVMDFQKVGENEWTKIGDDKCRL |
| 158 | D355N | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLINRHPEYRSLLLAVGGSGNGAMQMSTIGGFITDALEGHLQKELKHALRWRPEIATERDWKDTQNRFGGPNKVMDFQKVGENEWTKIGDDKCRL |
| 159 | D355Q | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIQRHPEYRSLLLAVGGSGNGAMQMSTIGGFITDALEGHLQKELKHALRWRPEIATERDWKDTQNRFGGPNKVMDFQKVGENEWTKIGDDKCRL |
| 160 | D355S | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLISRHPEYRSLLLAVGGSGNGAMQMSTIGGFITDALEGHLQKELKHALRWRPEIATERDWKDTQNRFGGPNKVMDFQKVGENEWTKIGDDKCRL |
| 161 | D355T | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLITRHPEYRSLLLAVGGSGNGAMQMSTIGGFITDALEGHLQKELKHALRWRPEIATERDWKDTQNRFGGPNKVMDFQKVGENEWTKIGDDKCRL |
| 162 | D355Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIYRHPEYRSLLLAVGGSGNGAMQMSTIGGFITDALEGHLQKELKHALRWRPEIATERDWKDTQNRFGGPNKVMDFQKVGENEWTKIGDDKCRL |
| 163 | D355C | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLICRHPEYRSLLLAVGGSGNGAMQMSTIGGFITDALEGHLQKELKHALRWRPEIATERDWKDTQNRFGGPNKVMDFQKVGENEWTKIGDDKCRL |
| 164 | D355G | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIGRHPEYRSLLLAVGGSGNGAMQMSTIGGFITDALEGHLQKELKHALRWRPEIATERDWKDTQNRFGGPNKVMDFQKVGENEWTKIGDDKCRL |
| 165 | D355A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIARHPEYRSLLLAVGGSGNGAMQMSTIGGFITDALEGHLQKELKHALRWRPEIATERDWKDTQNRFGGPNKVMDFQKVGENEWTKIGDDKCRL |
| 166 | D355V | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIVRHPEYRSLLLAVGGSGNGAMQMSTIGGFITDALEGHLQKELKHALRWRPEIATERDWKDTQNRFGGPNKVMDFQKVGENEWTKIGDDKCRL |
| 167 | D355P | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIPRHPEYRSLLLAVGGSGNGAMQMSTIGGFITDALEGHLQKELKHALRWRPEIATERDWKDTQNRFGGPNKVMDFQKVGENEWTKIGDDKCRL |
| 168 | D355L | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLILRHPEYRSLLLAVGGSGNGAMQMSTIGGFITDALEGHLQKELKHALRWRPEIATERDWKDTQNRFGGPNKVMDFQKVGENEWTKIGDDKCRL |
| 169 | D355I | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIIRHPEYRSLLLAVGGSGNGAMQMSTIGGFITDALEGHLQKELKHALRWRPEIATERDWKDTQNRFGGPNKVMDFQKVGENEWTKIGDDKCRL |
| 170 | D355F | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIFRHPEYRSLLLAVGGSGNGAMQMSTIGGFITDALEGHLQKELKHALRWRPEIATERDWKDTQNRFGGPNKVMDFQKVGENEWTKIGDDKCRL |

(continued)

| SEQ ID NO: | Variation | Amino Acid Sequence |
|---|---|---|
| **Amino Acid Sequence Identical to Amino Acid Sequence Set Forth in SEQ ID NO: 1 Except for Substitution of D355 with Another Amino Acid** | | |
| 171 | D355M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIMRHPEYRSLLLAVGGSGNGAMQMSTIGGFITDALEGHLQKELKHALRWRPEIATERDWKDTQNRFGGPNKVMDFQKVGENEWTKIGDDKCRL , |
| 172 | D355W | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIWRHPEYRSLLLAVGGSGNGAMQMSTIGGFITDALEGHLQKELKHALRWRPEIATERDWKDTQNRFGGPNKVMDFQKVGENEWTKIGDDKCRL , |

[Table 10]

**Amino Acid Sequence (1) Identical to Amino Acid Sequence Set Forth in SEQ ID NO: 1 Except for Substitution of Two Amino Acids with Other Amino Acids**

| SEQ ID NO: | Variation | Amino Acid Sequence |
|---|---|---|
| 173 | N5S, M58F | MASSSLLLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 174 | N5S, G225N | MASSSLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 175 | N5S, G225A | MASSSLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 176 | N5S, G440A | MASSSLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |
| 177 | N5S, G440M | MASSSLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 178 | E5L, M58F | MASSLLLLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 179 | E5L, G225N | MASSLLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 180 | E5L, G225A | MASSLLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 181 | E5L, K277Y | MASSLLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 182 | E5L, G440D | MASSLLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIDDDKCRL |
| 183 | N5L, G440A | MASSSLLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFFKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |

| Amino Acid Sequence (1) Identical to Amino Acid Sequence Set Forth in SEQ ID NO: 1 Except for Substitution of Two Amino Acids with Other Amino Acids | | |
|---|---|---|
| SEQ ID NO: | Variation | Amino Acid Sequence |
| 184 | N5L<br><br>G440M | MASSLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 185 | M58F<br><br>G225N | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

[Table 11]

| SEQ ID NO: | Variation | Amino Acid Sequence |
|---|---|---|
| 186 | M58F<br>G225A | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 187 | M58F<br>K277Y | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFKKQLRPTAWTLCHIKMTPEEAKKYRNLPVLFNVAKGFFMEPDEDYHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 188 | M58F<br>G440D | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIDDDKCRL |
| 189 | M58F<br>G440A | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |
| 190 | M58F<br>G440M | MASSNLTLTSSILIVGAGTWGCSTALHLARRGYKNVTVLDPHPVPSPIAAGNDINKIFEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 191 | G225N<br>G440A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |
| 192 | G225N<br>G440M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 193 | G225N<br>K277Y | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDYHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 194 | G225N<br>G440D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIDDDKCRL |
| 195 | G225A<br>G440A | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIADDKCRL |
| 196 | G225A<br>G440M | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAASDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIMDDKCRL |
| 197 | K277Y<br>G440D | MASSNLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGAGSDRLL<br>DFKKQLRPTAWTLCHIKMTPEEAKKYRNLPVLFNVAKGFFMEPDEDYHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIDDDKCRL<br>MASSLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |
| 198 | N5L<br>G225N | MASSLLTLTSSILIVGAGTWGCSTALHLARRGYKNVTVLDPHPVPSPIAAGNDINKIMEHREVKASETDPWSIAFSTCTRAALKGWKTDPVFQPYFHETGAIVSGHTPSLIKHIE<br>EHEIDSSDAEFVKLNTAEDFRKTMPPGILTGNFPGWKGWLNKTGAGWIHAKKAMFSAYTEAKRLGVTFITGSPEGDVVSLVYENGDVAGARTADGTIHRAEHTILSAGANSDRLL<br>DFKKQLRPTAWTLCHIKMTPEEAKKYRNLPVLFNVAKGFFMEPDEDKHELKICDEHPGYCNFIPDPKHGGEVRSIPFAKHQIPLEAEARARDFLRDTMPHLADRPLSFARICWDA<br>DTVDRAFLIDRHPEYRSLLLAVGGSGNGAMQMSTIGGFITDALEGHLQKELKHALRWRPEIATERDWKDTQNRFGGPNKVMDFQKVGENEWTKIGDDKCRL |

Other examples of the amino acid sequences of fructosyl amino acid oxidases of the present invention are shown. The amino acid sequences shown below are those that are identical to the amino acid sequence set forth in SEQ ID NO: 199 except that the methionine that is the amino acid at position 58 of SEQ ID NO: 199 is substituted with one selected from the group consisting of alanine, arginine, aspartic acid, cysteine, glutamine, glutamic acid, glycine, histidine, isoleucine, leucine, lysine, methionine, phenylalanine, proline, serine, threonine, tryptophan, tyrosine, and valine.

The amino acid sequence set forth in SEQ ID NO: 199 is that of a fructosyl amino acid oxidase (Accession Number AAB88209) derived from Aspergillus fumigatus, which is also referred to as "amadoriase I". The amino acid sequence set forth in SEQ ID NO: 199 was found to have an amino acid homology of 79.2% to the amino acid sequence set forth in SEQ ID NO: 1. The amino acid sequences set forth in SEQ ID NOs: 200 to 218, which were obtained by substituting one amino acid of the amino acid sequence set forth in SEQ ID NO: 199, were found to have an amino acid homology of 79.0% to the amino acid sequence set forth in SEQ ID NO: 1.

[Table 12]

| Amino Acid Sequence Identical to Amino Acid Sequence Set Forth in SEQ ID NO: 199 Except for Substitution of M58 with Another Amino Acid | | |
|---|---|---|
| SEQ ID NO: | Variation | Amino Acid Sequence |
| 200 | M58D | MAPSILSTESSIIVIGAGTWGCSTALHLARRGYKDVTVLDPHPVPSPIAAGNDINKIDEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 201 | M58E | MAPSILSTESSIIVIGAGTWGCSTALHLARRGYKDVTVLDPHPVPSPIAAGNDINKIEEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 202 | M58K | MAPSILSTESSIIVIGAGTWGCSTALHLARRGYKDVTVLDPHPVPSPIAAGNDINKIKEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 203 | M58R | MAPSILSTESSIIVIGAGTWGCSTALHLARRGYKDVTVLDPHPVPSPIAAGNDINKIREHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 204 | M58H | MAPSILSTESSIIVIGAGTWGCSTALHLARRGYKDVTVLDPHPVPSPIAAGNDINKIHEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 205 | M58N | MAPSILSTESSIIVIGAGTWGCSTALHLARRGYKDVTVLDPHPVPSPIAAGNDINKINEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 206 | M58Q | MAPSILSTESSIIVIGAGTWGCSTALHLARRGYKDVTVLDPHPVPSPIAAGNDINKIQEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 207 | M58S | MAPSILSTESSIIVIGAGTWGCSTALHLARRGYKDVTVLDPHPVPSPIAAGNDINKISEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 208 | M58T | MAPSILSTESSIIVIGAGTWGCSTALHLARRGYKDVTVLDPHPVPSPIAAGNDINKITEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 209 | M58Y | MAPSILSTESSIIVIGAGTWGCSTALHLARRGYKDVTVLDPHPVPSPIAAGNDINKIYEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 210 | M58C | MAPSILSTESSIIVIGAGTWGCSTALHLARRGYKDVTVLDPHPVPSPIAAGNDINKICEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 211 | M58G | MAPSILSTESSIIVIGAGTWGCSTALHLARRGYKDVTVLDPHPVPSPIAAGNDINKIGEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 212 | MS8A | MAPSILSTESSIIVIGAGTWGCSTALHLARRGYKDVTVLDPHPVPSPIAAGNDINKIAEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 213 | M58V | MAPSILSTESSIIVIGAGTWGCSTALHLARRGYKDVTVLDPHPVPSPIAAGNDINKIAEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP<br>MAPSILSTESSIIVIGAGTWGCSTALHLARRGYKDVTVLDPHPVPSPIAAGNDINKIVEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 214 | M58P | MAPSILSTESSIIVIGAGTWGCSTALHLARRGYKDVTVLDPHPVPSPIAAGNDINKIPEHSELKDGSSDPRSAAFSTFTRAALKAWKTDPVFQPYFHETGFIISGHTPALIDHIR KDEVEPSETNFVKLETAEDFRRTMPPGVLTGDFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |

(continued)

| Amino Acid Sequence Identical to Amino Acid Sequence Set Forth in SEQ ID NO: 199 Except for Substitution of M58 with Another Amino Acid | | |
|---|---|---|
| SEQ ID NO: | Variation | Amino Acid Sequence |
| 215 | H58L | MAPSILSTESSIIVIGAGTWGCSTALHLARRGYKDVTVLDPHPVPSPIAAGNDINKILEHSELKDGSSDPRSAAFSTFTRAALKAWKTDPVFQPYFHETGFIISGHTPALIDHIR KDEVEPSETNFVKLETAEDFRRTMPPGVLTGDFFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 216 | M58I | MAPSILSTESSIIVIGAGTWGCSTALHLARRGYKDVTVLDPHPVPSPIAAGNDINKIIEHSELKDGSSDPRSAAFSTFTRAALKAWKTDPVFQPYFHETGFIISGHTPALIDHIR KDEVEPSETNFVKLETAEDFRRTMPPGVLTGDFFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 217 | M58F | MAPSILSTESSIIVIGAGTWGCSTALHLARRGYKDVTVLDPHPVPSPIAAGNDINKIFEHSELKDGSSDPRSAAFSTFTRAALKAWKTDPVFQPYFHETGFIISGHTPALIDHIR KDEVEPSETNFVKLETAEDFRRTMPPGVLTGDFFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP |
| 218 | M58W | MAPSILSTESSIIVIGAGTWGCSTALHLARRGYKDVTVLDPHPVPSPIAAGNDINKIWEHSELKDGSSDPRSAAFSTFTRAALKAWKTDPVFQPYFHETGFIISGHTPALIDHIR KDEVEPSETNFVKLETAEDFRRTMPPGVLTGDFFPGWKGWLHKSGAGWIHAKKAMISAFNEAKRLGVRFVTGSPEGNVVSLVYEDGDVVGARTADGRVHKAHRTILSAGAGSDSLL DFKKQLRPTAWTLCHIQMGPEEVKQYRNLPVLFNIAKGFFMEPDEDKHELKICDEHPGYCNFLPDDNRPGQEKSVPFAKHQIPLEAEARARDFLHDTMPHLADRPLSFARICWDA DTPDRAFLIDRHPEHPSLLVAVGGSGNGAMQMPTIGGFIADALESKLQKEVKDIVRWRPETAVDRDWRATQNRFGGPDRIMDFQQVGEDQWTKIGESRGP _____ |

Another embodiment of the present invention is a fructosyl amino acid oxidase having an amino acid sequence that is identical to the amino acid sequence set forth in any of SEQ ID NOs listed above, except that one or more amino acids are modified or mutated, or deleted, substituted, added, and/or inserted, and the fructosyl amino acid oxidase has physicochemical properties such as high thermal stability and a high substrate specificity for a particular glycated amino acid or glycated peptide. The one or more amino acids are 1 to 15, 1 to 10, 1 to 9, 1 to 8, 1 to 7, 1 to 6, 1 to 5, 1 to 4, 1 to 3, or 1 or 2 amino acids.

Furthermore, the present invention provides a gene including a nucleotide sequence that encodes the amino acid sequences described above. The gene may be a DNA or an RNA and may be a complementary DNA (cDNA). Furthermore, the present invention also provides an expression vector containing a gene including a nucleotide sequence that encodes the amino acid sequences shown below.

A fructosyl amino acid oxidase of the present invention may be a recombinant protein obtained by transfecting, into a host cell, a nucleic acid including a nucleotide sequence that encodes the fructosyl amino acid oxidase of the present invention, to express the nucleotide sequence. Examples of the host cells include E. coli, yeasts, mammalian cells, and insect cells. The recombinant protein may be a recombinant protein synthesized in a cell-free protein synthesis system.

An embodiment of the present invention is a fructosyl amino acid oxidase including any of the amino acid sequences described above. In this embodiment, an amino acid sequence that serves as a tag may be attached to an N-terminus side and/or a C-terminus side to facilitate the purification and detection of enzymes. The tag may be, for example, a His tag, a Myc tag, an HA tag, a FLAG tag, or the like.

A fructosyl amino acid oxidase of the present invention has high activity for substrates while the fructosyl amino acid oxidase is immobilized on a support and/or is not immobilized on a support. In an embodiment of the present invention, a fructosyl amino acid oxidase produced by amino acid substitution as described above has higher activity for substrates than a fructosyl amino acid oxidase consisting of an amino acid sequence in which no amino acids are substituted. The fructosyl amino acid oxidase of the present invention may have an activity that is improved by an amount equal to or greater than 3%, 4%, 5%, 6%, 7%, 8%, 9%, 10%, 20%, 30%, 40%, 50%, 60%, 70%, 80%, 90%, or 100% of the activity of a fructosyl amino acid oxidase that has yet to undergo amino acid substitution. These values are values rounded to the nearest whole number.

An activity improvement ratio of the fructosyl amino acid oxidase of the present invention with respect to that of a wild-type fructosyl amino acid oxidase can be calculated, for example, according to the equation shown below. The calculated activity improvement ratio is a positive value in cases where the activity is improved as a result of the amino acid substitution, and the calculated activity improvement ratio is a negative value in cases where the activity is reduced as a result of the amino acid substitution.

[Math. 1]

$$\text{Activity improvement ratio (\%)} = \frac{\text{Enzymatic activity of amino-acid-substituted FAOD}}{\text{Enzymatic activity of wild-type FAOD}} \times 100 - 100$$

Another fructosyl amino acid oxidase of the present invention is a fructosyl amino acid oxidase having improved activity compared to a wild-type fructosyl amino acid oxidase, as measured after a heat treatment is performed on the fructosyl amino acid oxidase while it is immobilized on a support and/or is not immobilized on a support. The fructosyl amino acid oxidase of the present invention may have an activity exhibited after a heat treatment that is improved by an amount equal to or greater than 3%, 4%, 5%, 6%, 7%, 8%, 9%, 10%, 20%, 30%, 40%, 50%, 60%, 70%, 80%, 90%, or 100% of the activity of a fructosyl amino acid oxidase that has yet to undergo amino acid substitution. These values are values rounded to the nearest whole number (the same applies hereinafter).

Fructosyl amino acid oxidases of the present invention are stable to heat while they are immobilized on a support and/or are not immobilized on a support. In an embodiment of the present invention, a fructosyl amino acid oxidase produced by amino acid substitution as described above has higher thermal stability than a fructosyl amino acid oxidase consisting of an amino acid sequence in which no amino acids are substituted. Specifically, the fructosyl amino acid oxidase of the present invention may have a thermal stability that is improved by an amount equal to or greater than 3%, 4%, 5%, 6%, 7%, 8%, 9%, or 10% of the thermal stability of a fructosyl amino acid oxidase that has yet to undergo amino acid substitution. These values are values rounded to the nearest whole number (the same applies hereinafter).

A fructosyl amino acid oxidase consisting of the amino acid sequence set forth in SEQ ID NO: 1 is stable to heat while it is immobilized on a support. The "fructosyl amino acid oxidase that is stable to heat while it is immobilized on a support" is a fructosyl amino acid oxidase having a high residual activity after being heat-treated while being immobilized on a support. An example of such a fructosyl amino acid oxidase is one having a residual activity after a treatment at 65°C of 70% or greater.

More specifically, the temperature for the treatment may be 45°C, 48°C, 50°C, 55°C, 60°C, or 65°C. Furthermore, a duration of the treatment may be 10 to 30 minutes, preferably 15 to 25 minutes, and most preferably 20 minutes. The residual activity after heat treatment may be equal to or greater than 30%, 40%, 50%, 60%, or 70%.

More specifically, a fructosyl amino acid oxidase of an embodiment of the present invention is included in a glycated protein sensor. An example of the glycated protein sensor includes a fructosyl amino acid oxidase immobilized on a support. The fructosyl amino acid oxidase included in the glycated protein sensor has a very high residual activity after the protein sensor is heat-treated. Accordingly, even if the sensor is placed in a high-temperature environment, an output value of the sensor is maintained at a high level. Specifically, in the sensor of this embodiment, an output value of the sensor, which is a value after the sensor is allowed to stand at a temperature of 65°C or 80°C for 20 minutes, may be equal to or greater than 60% of an output value of the sensor, which is a value before the sensor is allowed to stand. Furthermore, an output value of the sensor, which is a value after the sensor is allowed to stand at a temperature of 75°C and a relative humidity of 60% for one day, two days, or three days, may be equal to or greater than 40%, 50%, 60%, or 70% of an output value of the sensor, which is a value before the sensor is allowed to stand.

Another example of the fructosyl amino acid oxidase that is stable to heat while it is immobilized on a support is a fructosyl amino acid oxidase having a residual activity after being heat-treated while being immobilized on a support, the residual activity being higher than a residual activity exhibited by the fructosyl amino acid oxidase after being treated under the same condition but without being immobilized on a support. More specifically, the temperature for the treatment is 45°C, 48°C, 50°C, 55°C, 60°C, or 65°C. Furthermore, the duration of the treatment is 10 to 30 minutes, preferably 15 to 25 minutes, and most preferably 20 minutes.

The thermal stability of a fructosyl amino acid oxidase can be determined by heating the support on which the fructosyl amino acid oxidase has been immobilized, by using a known means, such as an electric oven or a constant-temperature oven and then measuring the residual activity of the fructosyl amino acid oxidase. As referred to herein, the residual activity is a ratio of enzymatic activity exhibited after heat treatment at various temperatures to enzymatic activity exhibited before heat treatment.

The fructosyl amino acid oxidase of the present invention, which is produced by substituting an amino acid of the amino acid sequence set forth in SEQ ID NO: 1, has improved thermal stability compared to the fructosyl amino acid oxidase consisting of the amino acid sequence set forth in SEQ ID NO: 1. As demonstrated in Examples, the fructosyl amino acid oxidase consisting of the amino acid sequence set forth in SEQ ID NO: 1 has a very high thermal stability compared to other FAODs. Accordingly, even if the ratio of improvement in the thermal stability due to the amino acid substitution is several percent, the effect can be deemed to be significant.

As referred to herein, the thermal stability is, specifically, a thermal stability for a heat treatment performed at 45°C or 48°C for 15 minutes. The evaluation of the thermal stability of the fructosyl amino acid oxidases can be performed by any method. An example of the method is one in which the activity of a heat-treated FAOD and the activity of a non-heat-treated FAOD are measured to calculate the residual activity as the percentage of the enzymatic activity remaining after heat treatment.

Furthermore, in a case where the thermal stabilities of two FAODs are to be compared with each other, it suffices to compare their residual activities with each other. For example, the ratio of improvement in the thermal stability due to the amino acid substitution can be calculated according to the equation shown below. The calculated thermal stability improvement ratio is a positive value in cases where the thermal stability is improved as a result of the amino acid substitution, and the calculated thermal stability improvement ratio is a negative value in cases where the thermal stability is reduced as a result of the amino acid substitution.

[Math. 2]

$$\text{Thermal stability improvement ratio (\%)} = \frac{\text{Residual activity ratio of amino-acid-substituted FAOD (\%)}}{\text{Residual activity ratio of wild-type FAOD (\%)}} \times 100 - 100$$

In an embodiment of the present invention, a fructosyl amino acid oxidase is stable to heat while it is immobilized on a support. Exemplary means for immobilizing enzymes, such as FAODs, onto a support include covalent attachment methods, physical adsorption methods, ionic bonding methods, crosslinking methods, entrapment methods, and biochemical specific binding methods. An immobilizing method that does not cause deactivation of the enzyme may be selected in accordance with the enzyme that is used. Two or more immobilizing methods may be used in combination.

In some embodiments, the FAOD, alone or bound to a carrier, is immobilized onto a support by crosslinking induced by a crosslinking agent. In such cases, the support is a type of protein different from the FAOD, and the FAOD is immobilized onto the support by using a crosslinking agent, such as a glutaraldehyde or an isocyanate derivative, after mixing the FAOD with the support. Specific examples of the protein include albumins, such as bovine serum albumin (BSA), collagens, and gelatins.

The crosslinking agent to be used is a substance that undergoes inter- or intra-molecular crosslinking, and specific examples thereof include glutaraldehydes, isocyanate derivatives, formaldehydes, glyoxals, malondialdehydes, and succinaldehydes. In particular, it is preferable to use an amine-reactive crosslinking agent as the crosslinking agent. The amine-reactive crosslinking agent is a crosslinking agent that crosslinks proteins by reacting with the amino group of the proteins. Specific examples of the amine-reactive crosslinking agent include glutaraldehydes, formaldehydes, N-hydroxy esters, amide esters, and imide esters.

The amine-reactive crosslinking agent causes crosslinking by reacting with the amino group present on a surface of the fructosyl amino acid oxidase used in the present invention, and, accordingly, the fructosyl amino acid oxidase is stable to heat while it is immobilized on a support.

In other embodiments, the support may be a synthetic polymer, a resin, an inorganic material, a polysaccharide, a mineral, a clay, or the like. Specifically, the means for immobilizing the FAOD onto a support may be, for example, any of the following means: in cases where a fluororesin, an ion exchange resin, a polyvinyl alcohol resin, a water-curable resin, a photocurable resin, a solid polymer electrolyte, a polyion complex, a urethane resin, or the like is used as the support, the FAOD is immobilized onto the support; in cases where charcoal, bone charcoal, a silica gel, glass, a zeolite, a Celite, an alumina, titanium oxide, a ceramic, a hydroxyapatite, or the like is used as the support, the FAOD is immobilized onto the support by utilizing physical interaction between the support and the FAOD: in cases where a semipermeable membrane made of nylon, cellophane, ethyl cellulose, acetyl cellulose, a polystyrene, or the like or a phospholipid membrane is used as the support, the FAOD is immobilized onto the support with the membrane enclosing the FAOD; and in cases where a polyacrylamide gel, an agar, a gelatin, a carrageenan, a sodium alginate gel, a calcium alginate gel, a chitosan gel, or the like is used as the support, the FAOD is immobilized onto the gel.

The support may be a carbon material and/or beads. The carbon material may be a carbon nanotube, a fullerene, a graphene, or the like. The beads may be carbon microparticles (carbon beads), silica ($SiO_2$) microparticles (silica beads), or beads made of a polysaccharide, such as chitin, chitosan, or alginic acid. The beads may include metal microparticles or a magnetizable substance and may be magnetic beads. The beads may have an average particle size of 10 nm or greater and 200 nm or less. The FAOD can be immobilized onto the beads by being crosslinked onto the beads.

Furthermore, the present invention provides a glycated protein sensor. The glycated protein sensor of the present invention includes at least a support and an enzyme immobilized on the support. The enzyme includes at least a fructosyl amino acid oxidase. The glycated protein sensor of the present invention may be a glycated protein sensor that calculates an amount of a glycated protein present in a test sample, that is, a concentration of a glycated protein in a test sample.

Since the fructosyl amino acid oxidase included in the sensor of the present invention is stable to heat while it is immobilized on a support, the sensor has a feature of being able to provide highly reliable measurement results over a long period of time. In a case where the sensor of the present invention was allowed to stand at a temperature of 65°C or 80°C for 20 minutes, the sensor maintained an output value equal to or greater than 70% of the output value obtained before the sensor was allowed to stand. In a case where the sensor of the present invention was allowed to stand under the conditions of a temperature of 75°C and a relative humidity of 60% for one day and for two days, the sensor maintained output values of 48% and 29%, respectively, of the output value obtained before the sensor was allowed to stand.

A preferred embodiment of the glycated protein sensor of the present invention includes a support, a fructosyl amino acid oxidase immobilized on the support, and a detector. The detector detects hydrogen peroxide produced from a glycated amino acid and/or a glycated peptide by using the fructosyl amino acid oxidase. The glycated protein sensor can calculate the amount (concentration) of the glycated protein from the results of the detection performed by the detector.

In an embodiment of the glycated protein sensor of the present invention, the detector is a hydrogen peroxide detector that detects hydrogen peroxide, and specifically, the detector may be a hydrogen peroxide electrode. During the decomposition of the hydrogen peroxide into oxygen, electrons are emitted, and the hydrogen peroxide electrode detects the electrons as a current and, accordingly, can calculate the amount (concentration) of the hydrogen peroxide from the detected current value.

In another embodiment, the detector is an optical detector and detects hydrogen peroxide by measuring an absorbance or an amount of light. For example, the amount of hydrogen peroxide can be determined by causing a color reaction in the presence of a peroxidase and an oxidative coloring agent or detecting a luminol emission intensity.

In another embodiment, the detector is an electrochemiluminescence detector in which a gold electrode, a platinum electrode, or a transparent electrode made of indium tin oxide (ITO electrode) is used. Hydrogen peroxide is detected by measuring the emission from a luminescent reagent, such as luminol. The detector may be a hydrogen peroxide detector that uses a different method.

The test sample for the glycated protein sensor of the present invention may be a solution. The solution may be a body fluid, a solution that is body-fluid-derived, or a diluted solution of a body fluid. The solution may be a solution that is not a body fluid (not body-fluid-derived) or may be a liquid mixture of a body fluid or a solution that is body-fluid-derived and a solution that is not body-fluid-derived. The solution may be a solution for use in a measurement of samples or a solution for use in a measurement for calibration. For example, the solution may be a standard solution or a calibration solution. The solution may include a buffer solution.

The body fluid may be blood, a serum, a plasma, or a lymph fluid, may be a tissue fluid, such as an intercellular fluid, a transcellular fluid, or an interstitial fluid, or may be a body cavity fluid, a serous cavity fluid, a pleural fluid, an ascites fluid, a pericardial fluid, a cerebrospinal fluid (spinal fluid), a joint fluid (synovial fluid), or an eye's aqueous humor (aqueous humor). The body fluid may be a digestive fluid, such as saliva, gastric juice, bile, pancreatic juice, or an intestinal fluid, or may be sweat, tears, nasal mucus, urine, semen, a vaginal fluid, an amniotic fluid, or milk. The body fluid may be an animal body fluid or a human body fluid. The body fluid may be a liquid present in food containing an animal-derived protein (e.g., milk or a dairy product). The body fluid may be a plant body fluid, a plant biological fluid, or a plant-derived liquid. For example, the body fluid may be a plant juice, a nectar, or a sap.

The solution may contain a measurement target substance. For example, the solution may be tears, and the measurement target substance may be albumin or glycoalbumin present in the tears. The measurement target substance may be albumin, glycoalbumin, hemoglobin, or glycohemoglobin present in blood, a serum, or a plasma, may be albumin or glycoalbumin present in an interstitial fluid, may be albumin or glycoalbumin present in urine, or may be albumin or glycoalbumin present in saliva.

A measurement target of the glycated protein sensor of the present invention is a glycated protein. The glycated protein may be a fructosamine. The "fructosamine" is a general term for glycated proteins present in the blood. Specific examples thereof include glycated albumin and glycated hemoglobin present in blood.

An embodiment of the present invention may further include a protease immobilized on a support, in addition to the fructosyl amino acid oxidase. The "protease" is a general term for peptide bond hydrolases that hydrolyze and catabolize a protein or a polypeptide. The protease may be an enzyme that degrades a protein into peptide fragments. In cases where a protein contains a glycated amino acid residue, the protease acts to produce one or more selected from the group consisting of a glycated amino acid, a glycated-amino-acid-containing peptide fragment, a non-glycated amino acid, and a peptide fragment with no glycated amino acid. Among these, a glycated amino acid or a glycated-amino-acid-containing peptide fragment reacts with the fructosyl amino acid oxidase, and, consequently, hydrogen peroxide can be produced.

In an embodiment of the present invention, a support on which the enzyme is immobilized forms a support layer, which is, for example, a thin film layer, and the support layer is layered and positioned on a detection surface of the detector. The support is positioned on or near the detection surface of the detector by using a coupling agent, such as a silane coupling agent. The formation of a coupling layer between the layered support and the detector couples the support to the detection surface.

Furthermore, the present invention provides a method for measuring a glycated protein. The method of the present invention for measuring a glycated protein is a method for measuring a glycated protein including detecting a glycated protein by using a reaction of a fructosyl amino acid oxidase immobilized on a support. The measurement method of the present invention may be a non-diagnostic and/or diagnostic measurement method.

Furthermore, the present invention provides a method for producing a fructosyl amino acid oxidase. The method of the present invention for producing a fructosyl amino acid oxidase is a method for producing a fructosyl amino acid oxidase including an amino acid substitution step of substituting an amino acid of an amino acid sequence of the fructosyl amino acid oxidase, the amino acid being at a position corresponding to a position selected from the group consisting of positions 5, 58, 225, 277, and 440 of an amino acid sequence set forth in SEQ ID NO: 1, in a case where the amino acid sequence and the amino acid sequence set forth in SEQ ID NO: 1 are aligned with each other. In the fructosyl amino acid oxidase produced by the production method, an activity and/or thermal stability exhibited while it is immobilized on a support and/or is not immobilized on a support are higher than an activity and/or thermal stability of a fructosyl amino acid oxidase in which no amino acids are substituted.

The amino acid sequence used in the fructosyl amino acid oxidase has a high homology to the amino acid sequence set forth in SEQ ID NO: 1. Specifically, the amino acid sequence having a high homology to SEQ ID NO: 1 may include an amino acid sequence having a homology, to the amino acid sequence of SEQ ID NO: 1, of 30% or greater, 35% or greater, 40% or greater, 45% or greater, 50% or greater, 51% or greater, 52% or greater, 53% or greater, 54% or greater, 55% or greater, 56% or greater, 57% or greater, 58% or greater, 59% or greater, 60% or greater, 61% or greater, 62% or greater, 63% or greater, 64% or greater, 65% or greater, 66% or greater, 67% or greater, 68% or greater, 69% or greater, 70% or greater, 71% or greater, 72% or greater, 73% or greater, 74% or greater, 75% or greater, 76% or greater, 77% or greater, 78% or greater, 79% or greater, 80% or greater, 81% or greater, 82% or greater, 83% or greater, 84% or greater, 85% or greater, 86% or greater, 87% or greater, 88% or greater, 89% or greater, 90% or greater, 91% or greater, 92% or greater, 93% or greater, 94% or greater, 95% or greater, 96% or greater, 97% or greater, 98% or greater, or 99% or greater. These homology values are values rounded to the nearest whole number (the same applies hereinafter). The homology of amino acid sequences can be calculated, for example, by using default parameters in BLAST of the National Center for Biotechnology Information (NCBI) (http://www.ncbi.nlm.nih.gov/BLAST/).

EXAMPLES

The present invention will be described in more detail with reference to Examples. The present invention is not limited to the Examples described below.

1. Analysis of Thermal Stability and Substrate Specificity of Fructosyl Amino Acid Oxidase Having Amino Acid Sequence Set Forth in SEQ ID NO: 1

The thermal stability of two fructosyl amino acid oxidases exhibited while they were immobilized on a support was verified; one of them was a fructosyl amino acid oxidase containing amino acids set forth in SEQ ID NO: 1 and a His-tag attached to the C-terminus (hereinafter referred to as a "wild-type"), and the other was FAOD-E (manufactured by Kikkoman Corporation, hereinafter referred to as a "Comparative Example").

Glycated protein sensors were prepared with the enzyme of the wild-type and the enzyme of the Comparative Example. A silane coupling treatment was performed on a platinum electrode to incorporate an amino group into a surface of the electrode. In addition, bovine serum albumin (BSA) was added to a TES buffer solution, the enzymes were each added, and the solution was stirred. Furthermore, a predetermined amount of a glutaraldehyde solution was added to the mixed solution containing the enzyme and the BSA, and the solution was stirred. 1 μL of this solution was added dropwise to the platinum electrode having the amino group incorporated therein and was thoroughly dried. In this manner, sensors were prepared.

200 μL of a HEPES buffer solution containing 50 μM F-Lys was added dropwise to each of the sensors, and current output values were obtained. Each of the sensors was immersed in HEPES buffer solutions heated to respective temperatures of 50°C, 65°C, and 80°C, and the sensors were allowed to stand at the temperatures for 20 minutes. Subsequently, each of the sensors was allowed to stand at 4°C for 30 minutes so that an influence of residual heat could be eliminated. Output values for F-Lys were obtained in a manner similar to that of the measurement performed before the thermal treatment. A ratio of the output value after the thermal treatment to the output value before the thermal treatment was calculated as a rate of change in the output value, assuming that the output value before the thermal treatment was 100%.

The results of measuring the thermal stability of the immobilized enzymes are shown in Fig. 1. The sensor including the wild-type fructosyl amino acid oxidase (SEQ ID NO: 1) immobilized on a support maintained an output value of 70% or greater for F-Lys even after the sensor was allowed to stand at 65°C to 80°C for 20 minutes. Thus, the sensor showed superiority over the Comparative Example.

Furthermore, a substrate specificity of the wild-type and the Comparative Example was investigated in a liquid phase. The substrates used were fructosyl lysine (F-Lys), fructosyl valyl histidine (F-Val-His), fructosyl valine (F-Val), and fructosyl glycine (F-Gly). 20 μL of a substrate solution containing a 1 mM substrate was added to each of the wells of a microplate at room temperature; thereafter, 10 μL of a 0.5 wt.% oxidative colorimetric reagent TOOS (manufactured by Dojindo Laboratories), 260 μL of a solution containing 4.76 μg/mL of peroxidase and 0.1 mg/mL of 4-aminoantipyrine (manufactured by Nacalai Tesque, Inc.), a HEPES buffer solution (pH 8.0) containing 0.15 M sodium chloride, and 5 μL of an appropriately diluted solution of the fructosyl amino acid oxidase were added and mixed together; and thereafter, changes in an absorbance at 555 nm were measured under a condition of 37°C. A negative control used was a 20 mM potassium phosphate buffer (pH 7.5).

It was observed that the wild-type and the Comparative Example had a fructosyl amino acid oxidase activity in the case where F-Lys was used as the substrate, and, accordingly, the activity for each of the F-Val-His, F-Val, and F-Gly was measured under the same conditions as those used for the F-Lys. Specific activities for each of the F-Lys, F-Val-His, F-Val, and F-Gly of the wild-type and the Comparative Example are shown in the table below. The reactivities of the wild-type fructosyl amino acid oxidase (SEQ ID NO: 1) with F-Val-His, F-Val, and F-Gly were all less than 1 based on the reactivity with F-Lys, which was taken as 100. It was confirmed that the wild-type fructosyl amino acid oxidase had a specific reactivity with F-Lys.

[Table 13]

|  | F-Lys | F-Val-His | F-Val | F-Gly |
|---|---|---|---|---|
| Wild-Type (SEQ ID NO: 1 | 100 | 0.8 | 0.8 | 0.4 |
| Comparative Example | 100 | 0.0 | 54.0 | 7.0 |

2. Analysis of Activity and Thermal Stability of Fructosyl Amino Acid Oxidases of Examples Exhibited While They were not Immobilized on Support

The method for producing fructosyl amino acid oxidases for use in the Examples will be described in detail below. First, genes designed to include respective nucleotide sequences that encode the amino acid sequences described in this specification and to add a histidine tag to the N-terminus of each of the inserted proteins were transfected into respective expression vectors including a lactose operon. The expression vectors were used to transform an E. coli bacteria BL21 (DE3) Derived strain (manufactured by Nippon Gene Co., Ltd.).

Each of the transformed E. coli was cultured with shaking for 2 hours in an LB liquid medium containing 50 μg/mL of Kanamycin (manufactured by Nacalai Tesque, Inc.). In addition, these E. coli were cultured at 16°C for 20 hours in an LB liquid medium, which was supplemented with isopropyl β-D-thiogalactopyranoside (hereinafter referred to as "IPTG") to a final concentration of 0.1 mM. Then, the collected E. coli were suspended in a buffer A (20 mM Tris-HCL (pH 7.5) containing 0.25 M sodium chloride and 20 mM imidazole), and the bacteria were then sonicated in a sonicator and centrifuged to give a homogenate supernatant. The homogenate supernatant was treated with a filter having a pore size of 0.45 μm and subsequently was applied to EconoFit Nuvia IMAC Columns, Ni-charged (Bio-Rad Laboratories, Inc.) and washed with the buffer A in an amount seven times the volume of the column. Furthermore, elution was performed in an elution buffer (20 mM Tris-HCL (pH 7.5) containing 0.25 M sodium chloride and 0.5 M imidazole).

The resulting eluate was dialyzed against a 10 mM sodium phosphate buffer (pH 8.4) containing 150 mM sodium chloride and 50 vol.% glycerol, to give a sample. The protein concentration of the sample was measured with an absorbance at 280 nm.

Regarding the FAODs produced by substituting an amino acid of the amino acid sequence set forth in SEQ ID NO: 1 (hereinafter also referred to as "Examples") and a FAOD including the amino acid sequence set forth in SEQ ID NO: 1 (hereinafter referred to as a "wild-type"), their activity and thermal stability were evaluated. HEPES buffer solutions (hereinafter referred to as "diluted enzyme solutions") containing 0.01 to 0.1 mg/mL of the respective enzymes were each dispensed in an amount of 50 μL into tubes, and the tubes were held for 15 minutes on ice or on a heat block such that a temperature of 45°C could be achieved. Next, at room temperature, a 0.5 w/w% TOOS and a POD/4-AA solution (solution diluted with a HEPES buffer solution (pH 8.0, 0.15 M NaCl) such that the peroxidase was present in an amount of 4.76 μg/mL, and the 4-aminoantipyrine was present in an amount of 0.1 mg/mL) were added to the wells of a microtiter plate containing 1 mM F-Lys, and the diluted enzyme solution, which had been held on ice or heat-treated with the heat block, was also added thereto. These were mixed by pipetting. The absorbance at 555 nm was measured with a microtiter plate reader at a setting of 37°C, every 80 seconds for 20 minutes after the addition, and a change in the absorbance per unit time was calculated as a FAOD activity. Hereinafter, the activity measured from the enzyme that had been held on ice will be referred to as a "FAOD activity before heat treatment", and the activity measured from the enzyme that had been heat-treated with the heat block will be referred to as a "FAOD activity after heat treatment".

Activity improvement ratios before and after heat treatment and a thermal stability improvement ratio were calculated from average values of the FAOD activities of the Examples and the wild-type according to the equations below.

[Math. 3]

$$\text{Activity improvement ratio before heat treatment (\%)} = \frac{\text{FAOD activity of Example before heat treatment}}{\text{FAOD activity of wild-type before heat treatment}} \times 100 - 100$$

[Math. 4]

$$\text{Activity improvement ratio after heat treatment (\%)} = \frac{\text{FAOD activity of Example after heat treatment}}{\text{FAOD activity of wild-type after heat treatment}} \times 100 - 100$$

[Math. 5]

$$\text{Thermal stability improvement ratio (\%)} = \frac{\text{FAOD activity of Example after heat treatment / FAOD activity of Example before heat treatment}}{\text{FAOD activity of wild-type after heat treatment / FAOD activity of wild-type before heat treatment}} \times 100 - 100$$

Regarding the enzymes of the Examples, their activity improvement ratios before and after heat treatment and thermal stability improvement ratios are shown below. It was observed that the enzymes of the Examples had at least one of the following effects: improved activity before heat treatment, improved activity after heat treatment, and improved thermal stability, compared to the enzyme of the wild-type.

[Table 14]

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| N5S | 117.5 | 195.8 | 36.0 |
| N5Y | 37.4 | 47.3 | 7.2 |
| N5C | 13.1 | 66.4 | 47.1 |
| N5P | 8.2 | -31.9 | -37.0 |
| N5L | 55.2 | 97.4 | 27.2 |
| N5M | -7.0 | 8.7 | 17.0 |
| N5W | 4.2 | 48.1 | 42.2 |
| M58H | 7.7 | - | - |
| M58S | 7.3 | - | - |
| M58T | 50.4 | - | - |
| M58G | 23.9 | - | - |
| M58A | 11.1 | - | - |
| M58V | 74.4 | - | - |
| M58L | 74.5 | - | - |
| M581 | 68.8 | - | - |
| M58F | -35.7 | 197.6 | 363.0 |
| G225D | 14.0 | 68.9 | 48.1 |

(continued)

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| G225E | 392.7 | 95.9 | 376.6 |
| G225N | 338.4 | 180.0 | 609.3 |
| G225Q | 96.3 | 105.2 | 101.3 |
| G225S | 31.4 | 111.0 | 34.9 |
| G225T | 108.5 | 128.6 | 139.5 |
| G225Y | 465.0 | 70.4 | 327.5 |
| G225C | 104.2 | 81.9 | 85.4 |
| G225A | 236.7 | 162.7 | 385.1 |
| G225V | 75.4 | 138.8 | 104.6 |
| G225P | 158.7 | 144.8 | 229.8 |
| G225L | 218.9 | 85.9 | 188.0 |
| G225I | 168.1 | 87.9 | 147.8 |
| G225F | 226.5 | 94.3 | 213.5 |
| G225M | 225.3 | 89.8 | 202.4 |
| K277N | -49.0 | 50.8 | 195.6 |
| K277Y | 450.3 | 72.4 | 326.1 |
| K277A | 129.4 | 87.8 | 113.6 |
| K277F | 398.1 | 49.6 | 197.3 |
| K277W | 230.5 | 44.6 | 102.8 |
| K277L | 185.8 | 49.8 | 92.5 |
| G440E | -16.3 | -8.1 | 9.8 |
| G440S | 14.0 | 105.3 | 14.8 |

In the table, "-" indicates that no enzymatic activity after heat treatment was observed, and, therefore, that the value could not be calculated.

Furthermore, an Example including a plurality of amino acid substitutions was prepared based on the above results, and its enzymatic activity and thermal stability were evaluated. The prepared Example was a variant including two substituted amino acids, N5L and G225N, with respect to the amino acid sequence set forth in SEQ ID NO: 1.

The method of evaluation was as described above.

As shown below, it was observed that the activity after heat treatment and the thermal stability were significantly improved compared to variants including a single amino acid substitution, such as N5L or G225N.

[Table 15]

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| N5L + G225N | 128.5 | 674.5 | 239.0 |

Furthermore, in other Examples, the activity and the thermal stability exhibited without immobilization on a support were evaluated. The method of evaluation was as described above, and the heat treatment was performed at 45°C. Regarding the enzymes of the Examples, their activity improvement ratios after heat treatment are shown below. It was observed that the activity of the enzymes of the Examples after heat treatment was improved compared to the enzyme of the wild-type.

[Table 16]

| Amino Acid Substitution of Example | Activity Improvement Ratio After Heat Treatment (%) | Amino Acid Substitution of Example | Activity Improvement Ratio After Heat Treatment (%) |
|---|---|---|---|
| N5D | 58.1 | G225K | 28.6 |
| N5E | 44.2 | G440D | 129.5 |
| N5A | 3.9 | G440K | 46.1 |
| N5F | 242.3 | G440N | 11.2 |

Furthermore, variants of a different FAOD were prepared, and an investigation was made as to whether their activity and thermal stability were improved. The different FAOD used was a FAOD including an amino acid sequence set forth in SEQ ID NO: 199. This FAOD was a fructosyl amino acid oxidase (Accession Number AAB88209) derived from Aspergillus fumigatus, which is also referred to as "amadoriase I". The amino acid sequence of this FAOD was found to have an amino acid homology of 79.2% to the amino acid sequence set forth in SEQ ID NO: 1.

The FAOD and its variants were prepared with the same method as that described above, and their activity improvement ratio before heat treatment, activity improvement ratio after heat treatment, and thermal stability were evaluated. As shown in Table 17, it was observed that the variants obtained by substituting the methionine at position 58 with another amino acid had at least one of the following effects: improved activity before heat treatment, improved activity after heat treatment, and improved thermal stability, compared to the enzyme of the wild-type.

[Table 17]

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| M58F | -63.11 | 41.5 | 283.5 |
| M58A | 11.63 | - | - |
| M58I | 28.70 | 12.9 | -12.3 |

3. Analysis of Activity and Thermal Stability of Fructosyl Amino Acid Oxidases of Examples Exhibited While They Were Immobilized on Support

Regarding the fructosyl amino acid oxidases of the Examples and the wild-type, their activity and thermal stability exhibited while they were immobilized on a support were evaluated. The support used was bovine serum albumin (BSA). Hepes buffers (10 mM Hepes + 150 mM NaCl, pH 8.0) each containing 0.1 mg/mL of an enzyme and 0.264% (w/v) BSA were adjusted, and glutaraldehyde was added thereto such that it was present in a concentration of 0.1% (w/v). Incubation was performed at 25°C for 20 minutes, 90 μL of an ice-cold TAE buffer (40 mM Tris-acetate, 1 mM EDTA, pH 8.0 to 8.5) was added, and an excess amount of primary-amine-containing Tris was added to terminate the reaction. The resultant was held at 15°C on ice or at 45°C for 15 minutes on a heat block, and subsequently, the enzymatic activity was measured. The activity improvement ratios before and after heat treatment and the thermal stability improvement ratio were calculated from average values of the FAOD activities of each of the enzymes.

Regarding the enzymes of the Examples, their activity improvement ratios before and after heat treatment and thermal stability improvement ratio are shown below. It was observed that the enzymes of the Example that were immobilized on a support had at least one of the following effects: improved activity before heat treatment, improved activity after heat treatment, and improved thermal stability, compared to the enzyme of the wild-type.

[Table 18]

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| N5D | 11.6 | 1.1 | -9.4 |
| N5K | 15.3 | -1.4 | -14.5 |
| N5H | 7.8 | -5.5 | -12.4 |
| N5S | 176.0 | 177.5 | 0.5 |

(continued)

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| N5Y | 61.3 | 49.4 | -7.4 |
| N5C | 19.0 | 18.2 | -0.6 |
| N5G | 12.0 | 4.1 | -7.0 |
| N5A | 49.6 | 41.4 | -5.5 |
| N5V | 4.8 | -2.8 | -7.3 |
| N5P | 22.0 | 13.0 | -7.4 |
| N5L | 56.0 | 44.8 | -7.2 |
| N5F | 13.2 | 5.5 | -6.8 |
| N5M | 4.6 | -0.3 | -4.7 |
| N5W | 30.6 | 27.7 | -2.3 |
| M58T | 21.7 | -10.1 | -26.2 |
| M58G | 13.2 | -15.0 | -24.8 |
| M58A | -5.4 | 0.5 | 6.1 |
| M58V | 46.9 | 59.6 | 8.7 |
| M58L | 61.2 | 56.8 | -2.7 |
| M58I | 31.6 | 33.0 | 1.0 |
| M58F | -48.4 | -31.5 | 32.9 |
| G225D | -20.2 | -11.6 | 10.9 |
| G225E | -19.9 | -11.7 | 10.3 |
| G225H | -7.4 | -0.4 | 7.6 |
| G225N | 67.6 | 92.0 | 14.6 |
| G225Q | -19.1 | -9.3 | 12.2 |
| G225S | 2.5 | 6.6 | 4.0 |
| G225T | 22.3 | 30.5 | 6.7 |
| G225Y | -29.8 | -19.4 | 14.8 |
| G225C | -33.5 | -26.2 | 10.9 |
| G225A | 77.0 | 101.5 | 13.9 |
| G225V | 7.8 | 12.5 | 4.4 |
| G225P | 34.3 | 50.3 | 11.9 |
| G225L | -29.2 | -21.5 | 10.9 |

Furthermore, in other Examples, the activity and the thermal stability exhibited with immobilization on a support were evaluated. The method of evaluation was as described above, and the heat treatment was performed at 45°C. Regarding the enzymes of the Examples, their activity improvement ratios before and after heat treatment and thermal stability improvement ratio are shown below. It was observed that the enzymes of the Example that were immobilized on a support had at least one of the following effects: improved activity before heat treatment, improved activity after heat treatment, and improved thermal stability, compared to the enzyme of the wild-type.

[Table 19]

| Amino Acid Substitution of Example | Activity Improvement Ratio Before Heat Treatment (%) | Activity Improvement Ratio After Heat Treatment (%) | Thermal Stability Improvement Ratio (%) |
|---|---|---|---|
| G440E | 30.8 | 37.0 | 4.7 |
| G440K | 11.3 | 30.4 | 17.2 |
| G440N | -10.2 | 4.1 | 16.0 |
| G440Y | 21.3 | 26.2 | 4.0 |
| G440A | 36.5 | 56.0 | 14.3 |

Furthermore, in addition to the Examples and the wild-type, a Comparative Example (FAOD-E, manufactured by Kikkoman Corporation) was evaluated for its activity and thermal stability exhibited while it was immobilized on a support. The support used was bovine serum albumin (BSA), and the activity was measured in the manner described above. The heat treatment temperature was 45°C. A residual activity ratio after heat treatment and the thermal stability improvement ratio were calculated according to the equations below.

[Math. 6]

$$\text{Residual activity ratio after heat treatment (\%)} = \frac{\text{FAOD activity after heat treatment}}{\text{FAOD activity before heat treatment}} \times 100$$

[Math. 7]

$$\text{Thermal stability improvement ratio (\%)} = \frac{\text{Residual activity ratio of Example after heat treatment}}{\text{Residual activity ratio of wild-type after heat treatment}} \times 100 - 100$$

The table below shows the residual activity ratio of the Comparative Example and the wild-type and the residual activity ratio and the thermal stability improvement ratio of the Examples. In all of the Examples, a significant improvement in the thermal stability was observed compared to the wild-type.

[Table 20]

| | FAOD | Residual Activity Ratio After Heat Treatment (%) | Thermal Stability Improvement Ratio (%) |
|---|---|---|---|
| | Comparative Example | 8.1 | - |
| | Wild-Type (SEQ ID NO: 1) | 55.1 | - |
| Example | M58A | 64.5 | 17.0 |
| | M58F | 80.5 | 46.1 |
| | M58I | 56.9 | 3.3 |
| | G225N | 74.0 | 34.3 |
| | G225Y | 80.7 | 46.5 |
| | G225S | 66.6 | 20.8 |

4. Evaluation of Sensors Including Wild-Type or Variant Fructosyl Amino Acid Oxidase

A glycated protein sensor including a wild-type FAOD and a glycated protein sensor including a variant FAOD were prepared, and their thermal stability was evaluated. The wild-type FAOD used was a FAOD having the amino acid sequence set forth in SEQ ID NO: 1. The variant FAOD used was a FAOD that was identical to the wild-type FAOD except for a substituted amino acid M58F; that is, the variant FAOD had the amino acid sequence set forth in SEQ ID NO: 38. A silane coupling treatment was performed on a platinum electrode to incorporate an amino group into a surface of the electrode. The FAODs were each added to a TES buffer solution containing bovine serum albumin (BSA) dissolved therein. The solution was then stirred for dissolution and, further, was centrifugally filtered to remove impurities, to give an enzyme solution. A predetermined amount of a glutaraldehyde solution was added to the enzyme solution, which was then quickly stirred. 1 μL of this solution was added dropwise to the platinum electrode having the amino group incorporated therein and was thoroughly dried. In this manner, sensors including a FAOD and BSA, which was a support, were obtained. Four sensors were produced with the wild-type FAOD, and four sensors were produced with the variant FAOD. Thus, eight sensors in total were obtained.

200 μL of a HEPES buffer solution containing 50 μM F-Lys was added dropwise to each of the sensors, and changes in the obtained current value were recorded as the output value of the sensor. Each of the sensors used was washed with a HEPES buffer solution, and then water was removed with a blower. The sensors were allowed to stand under the conditions of a temperature of 75°C and a relative humidity of 60%. After a certain period of time elapsed, the sensors were taken out, and the output values of the sensors were recorded in a similar manner. This repetitive operation of allowing the sensor to stand and recording the output value was performed on each of the sensors on the first, second, third, sixth, and seventh days, and the output value on each of the days was recorded. The evaluation results are shown in Fig. 2. It is demonstrated that the sensors including a variant FAOD had significantly high output values of the first to seventh days compared to the sensors including a wild-type FAOD, and that their output values were also significantly high in terms of a rate of change, where the value of the 0th day was taken as 100%, for the first to seventh days.

Specifically, the sensors of the Examples, which are sensors including a support and a variant FAOD immobilized on the support, maintained an output value of 73% after the sensors were allowed to stand under conditions of 75°C and a relative humidity of 60% for one day, maintained an output value of 61% after the sensors were allowed to stand under the same conditions for two days, maintained an output value of 49% after the sensors were allowed to stand under the same conditions for three days, and maintained an output value of 27% after the sensors were allowed to stand under the same conditions for six to seven days.

While several embodiments and examples of the present disclosure have been described above, these embodiments and examples illustratively describe the present disclosure. For example, the above embodiments are those described in detail so that the present disclosure can be clearly described, and, if necessary, additional modifications may be made to dimensions, configurations, materials, and circuits. Note that the scope of the present disclosure encompasses embodiments in which one or more of the above-described features of the present disclosure are desirably combined. It is intended that the appended claims cover numerous modifications to the embodiments within the technical spirit of the present disclosure. Accordingly, it is to be understood that the embodiments and examples disclosed herein have been presented by way of illustration and should not be considered as limiting the scope of the present disclosure.

**Claims**

1.    A fructosyl amino acid oxidase comprising an amino acid sequence identical to an amino acid sequence set forth in SEQ ID NO: 1 except for an amino acid that is substituted, the amino acid being at a position corresponding to a position selected from the group consisting of positions 5, 58, 59, 98, 225, 277, 285, 355, and 440 of the amino acid sequence set forth in SEQ ID NO: 1, in a case where the amino acid sequence and the amino acid sequence set forth in SEQ ID NO: 1 are aligned with each other.

2.    The fructosyl amino acid oxidase according to claim 1, wherein the fructosyl amino acid oxidase has a higher fructosyl amino acid oxidase activity and/or thermal stability than a fructosyl amino acid oxidase in which no amino acids are substituted.

3.    The fructosyl amino acid oxidase according to claim 2, wherein the fructosyl amino acid oxidase has a 4% or more higher fructosyl amino acid oxidase activity and/or thermal stability than the fructosyl amino acid oxidase in which no amino acids are substituted.

4.    The fructosyl amino acid oxidase according to claim 2, wherein the activity and/or thermal stability is an activity and/or a thermal stability exhibited while the fructosyl amino acid oxidase is immobilized on a support.

5.    The fructosyl amino acid oxidase according to claim 1, wherein the fructosyl amino acid oxidase has an activity exhibited after heat treatment of the fructosyl amino acid oxidase, and the activity is higher than an activity of a fructosyl amino acid oxidase in which no amino acids are substituted, which is an activity exhibited after the heat treatment of the fructosyl amino acid oxidase in which no amino acids are substituted.

6.    The fructosyl amino acid oxidase according to claim 5, wherein conditions for the heat treatment include a temperature of 40°C or 48°C and a duration of 15 minutes.

7. The fructosyl amino acid oxidase according to claim 1, wherein the fructosyl amino acid oxidase has a higher thermal stability for a heat treatment performed at a temperature of 40°C or 48°C for a duration of 15 minutes than a fructosyl amino acid oxidase that has yet to undergo amino acid substitution.

8. The fructosyl amino acid oxidase according to any one of claims 1 to 7, wherein the fructosyl amino acid oxidase has a thermal stability exhibited while the fructosyl amino acid oxidase is immobilized on a support, and the thermal stability is higher than a thermal stability of the fructosyl amino acid oxidase exhibited while the fructosyl amino acid oxidase is not immobilized on a support.

9. The fructosyl amino acid oxidase according to claim 8, wherein the fructosyl amino acid oxidase has a specific reactivity with fructosyl lysine.

10. The fructosyl amino acid oxidase according to claim 9, wherein, regarding the reactivity, the fructosyl amino acid oxidase has reactivities with fructosyl valine, a fructosyl valine-containing peptide, and fructosyl glycine, and each of the reactivities is 20 or less based on the reactivity with fructosyl lysine, which is taken as 100.

11. The fructosyl amino acid oxidase according to claim 2, wherein the fructosyl amino acid oxidase comprises an amino acid sequence that shows a homology of 75% or greater to the amino acid sequence set forth in SEQ ID NO: 1.

12. The fructosyl amino acid oxidase according to claim 11, wherein the fructosyl amino acid oxidase comprises an amino acid sequence that satisfies at least one of (1) to (9) below:

(1) the amino acid at a position corresponding to position 5 of the amino acid sequence set forth in SEQ ID NO: 1 is one selected from the group consisting of alanine, arginine, aspartic acid, cysteine, glutamine, glutamic acid, glycine, histidine, leucine, methionine, phenylalanine, proline, serine, threonine, tryptophan, tyrosine, and valine;
(2) the amino acid at a position corresponding to position 58 of the amino acid sequence set forth in SEQ ID NO: 1 is one selected from the group consisting of alanine, arginine, asparagine, aspartic acid, cysteine, glutamine, glutamic acid, glycine, histidine, isoleucine, leucine, lysine, phenylalanine, proline, serine, threonine, tryptophan, tyrosine, and valine;
(3) the amino acid at a position corresponding to position 59 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;
(4) the amino acid at a position corresponding to position 98 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;
(5) the amino acid at a position corresponding to position 225 of the amino acid sequence set forth in SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine;
(6) the amino acid at a position corresponding to position 277 of the amino acid sequence set forth in SEQ ID NO: 1 is one selected from the group consisting of alanine, arginine, aspartic acid, cysteine, glutamine, glutamic acid, glycine, histidine, isoleucine, leucine, methionine, phenylalanine, proline, serine, threonine, tryptophan, tyrosine, and valine;
(7) the amino acid at a position corresponding to position 285 of the amino acid sequence set forth in SEQ ID NO: 1 is one selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, and valine;
(8) the amino acid at a position corresponding to position 355 of the amino acid sequence set forth in SEQ ID NO: 1 is one selected from the group consisting of alanine, arginine, asparagine, cysteine, glutamine, glutamic acid, glycine, histidine, isoleucine, leucine, lysine, methionine, phenylalanine, proline, serine, threonine, tryptophan, tyrosine, and valine; and
(9) the amino acid at a position corresponding to position 440 of the amino acid sequence set forth in SEQ ID NO: 1 is one selected from the group consisting of alanine, arginine, asparagine, aspartic acid, cysteine, glutamine, glutamic acid, histidine, isoleucine, leucine, lysine, methionine, phenylalanine, proline, serine, threonine, tryptophan, tyrosine, and valine.

13. A fructosyl amino acid oxidase comprising an amino acid sequence that shows a homology of 79% or greater to an amino acid sequence set forth in SEQ ID NO: 1 and in which an amino acid is histidine, serine, threonine, glycine, alanine, valine, leucine, isoleucine, or phenylalanine, the amino acid being at a position corresponding to position 58 of the amino acid sequence set forth in SEQ ID NO: 1 in a case where the amino acid sequence and the amino acid sequence set forth in SEQ ID NO: 1 are aligned with each other.

14. The fructosyl amino acid oxidase according to claim 13, wherein the amino acid is phenylalanine, alanine, or isoleucine.

15. The fructosyl amino acid oxidase according to claim 2, wherein the fructosyl amino acid oxidase comprises an amino acid sequence set forth in one of SEQ ID NOs: 2 to 198 and 200 to 218 or comprises another amino acid sequence that is identical to the amino acid sequence except that one to five amino acids are deleted, substituted, added, and/or inserted.

16. A gene encoding the amino acid sequence of the fructosyl amino acid oxidase according to any one of claims 11 to 15.

17. An expression vector comprising the gene according to claim 16.

18. A glycated protein sensor comprising the fructosyl amino acid oxidase according to claim 2 and a support on which the fructosyl amino acid oxidase is immobilized.

19. The glycated protein sensor according to claim 18, wherein the fructosyl amino acid oxidase is immobilized on the support as a result of crosslinking induced by an amine-reactive crosslinking agent.

20. The sensor according to claim 19, wherein an output value of the sensor, which is a value after the sensor is allowed to stand at a temperature of 65°C or 80°C for 20 minutes, is equal to or greater than 60% of an output value of the sensor, which is a value before the sensor is allowed to stand, or an output value of the sensor, which is a value after the sensor is allowed to stand at a temperature of 75°C and a relative humidity of 60% for one day, is equal to or greater than 40% of an output value of the sensor, which is a value before the sensor is allowed to stand.

21. A method for measuring a glycated protein, comprising detecting a glycated protein by using a reaction of the fructosyl amino acid oxidase according to claim 2.

22. A method for producing a fructosyl amino acid oxidase, comprising an amino acid substitution step of substituting an amino acid of an amino acid sequence of the fructosyl amino acid oxidase, the amino acid being at a position corresponding to a position selected from the group consisting of positions 5, 58, 225, 277, and 440 of an amino acid sequence set forth in SEQ ID NO: 1, in a case where the amino acid sequence and the amino acid sequence set forth in SEQ ID NO: 1 are aligned with each other.

23. The method for producing a fructosyl amino acid oxidase according to claim 22, wherein the amino acid sequence of the fructosyl amino acid oxidase is an amino acid sequence having a homology of 79% or greater to the amino acid sequence set forth in SEQ ID NO: 1.

[FIG. 1]

RATE OF CHANGE IN OUTPUT VALUE

Bar: S.E.

[FIG. 2]

**EP 4 748 936 A1**

## INTERNATIONAL SEARCH REPORT

| International application No. |
|---|
| **PCT/JP2024/025602** |

### A. CLASSIFICATION OF SUBJECT MATTER

*C12N 15/53*(2006.01)i; *C12N 9/06*(2006.01)i; *C12N 11/02*(2006.01)i; *C12N 15/63*(2006.01)i; *C12Q 1/00*(2006.01)i;
*C12Q 1/26*(2006.01)i
FI:   C12N15/53; C12N15/63 Z; C12N11/02; C12Q1/26; C12Q1/00 C; C12N9/06 ZNA

According to International Patent Classification (IPC) or to both national classification and IPC

### B. FIELDS SEARCHED

Minimum documentation searched (classification system followed by classification symbols)

C12N15/53; C12N9/06; C12N11/02; C12N15/63; C12Q1/00; C12Q1/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X | JP 2013-500729 A (F. HOFFMANN-LA ROCHE AG) 10 January 2013 (2013-01-10) fig. 1, paragraphs [0049]-[0060] | 1-10, 12, 16, 17 |
| Y | | 1-23 |
| X | KIM, Seung-Su, et al. Cumulative effect of amino acid substitution for the development of fructosyl valine-specific fructosyl amine oxidase. Enzyme and Microbial Technology. 2009, vol. 44, pp. 52-56 fig. 1, tables 1-3 | 1-10, 12, 16, 17 |
| Y | | 1-23 |
| X | FUJIWARA, Maki, et al. Alteration of Substrate Specificity of Fructosyl-Amino Acid Oxidase from Ulocladium sp. JS-103. Journal of Bioscience and Bioengineering. 2006, vol. 102, no. 3, pp. 241-243 abstract, fig. 2, tables 1-2 | 1-10, 12, 16, 17 |
| Y | | 1-23 |

✓ Further documents are listed in the continuation of Box C.   ✓ See patent family annex.

| | |
|---|---|
| \*    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | "&"   document member of the same patent family |
| "P"   document published prior to the international filing date but later than the priority date claimed | |

| Date of the actual completion of the international search | Date of mailing of the international search report |
|---|---|
| **06 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2024/025602** |

### C.    DOCUMENTS CONSIDERED TO BE RELEVANT

| Category* | Citation of document, with indication, where appropriate, of the relevant passages | Relevant to claim No. |
|---|---|---|
| X | FUJIWARA, Maki et al. Alteration of substrate specificity of fructosyl-amino acid oxidase from Fusarium oxysporum. Appl. Microbiol. Biotechnol. 2007, vol. 74, pp. 813-819 <br>     abstract, fig. 1, tables 1-2 | 1-10, 12, 16, 17 |
| Y |  | 1-23 |
| X | fructosyl amine:oxygen oxidoreductase [Aspergillus fumigatus]. GenBank, retrieved from the Internet. 1997, Acc. AAB88209.1, [retrieved on 06 August 2024], <URL: https://www.ncbi.nl m.nih.gov/protein/2661130?sat=4&satkey=39281210> <br>     FEATURES, ORIGIN | 1-11, 16, 17 |
| Y |  | 1-23 |
| X | WO 2015/060431 A1 (KIKKOMAN CORPORATION) 30 April 2015 (2015-04-30) <br>     fig. 1-1 to 1-5, paragraphs [0018]-[0019] | 1-10, 12, 16, 17 |
| Y |  | 1-23 |
| Y | ZHENG, Jing et al. Engineered amadoriase II exhibiting expanded substrate range. Appl. Microbiol. Biotechnol. 2010, vol. 86, pp. 607-613 <br>     abstract, table 1, fig. 2 | 1-23 |
| Y | MIURA, Seiji et al. Development of fructosyl amine oxidase specific to fructosyl valine by site-directed mutagenesis. Protein Engineering, Design & Selection. 2008, vol. 21, no. 4, pp. 233-239 <br>     abstract, tables 1-3 | 1-23 |
| Y | MIURA, Seiji et al. Active site analysis of fructosyl amine oxidase using homology modeling and site-directed mutagenesis. Biotechnol. Lett. 2006, vol. 28, pp. 1895-1900 <br>     abstract, fig. 1-2, tables 1-2 | 1-23 |
| Y | SAKAUE, Ryoichi et al. Thermostabilization of Bacterial Fructosyl-Amino Acid Oxidase by Directed Evolution. Appl. Environ. Microbiol. 2003, vol. 69, no. 1, pp. 139-145 <br>     abstract, fig. 1 | 1-23 |
| Y | WO 2019/221264 A1 (PROVIGATE INC.) 21 November 2019 (2019-11-21) <br>     claims 1, 38, example 3, paragraph [0177] | 1-23 |
| A | FAD dependent oxidoreductase [Aspergillus pseudotamarii]. NCBI Reference Sequence, retrieved from the Internet. 2020, Acc. XP_031920077.1, [retrieved on 06 August 2024], <URL: https://www.ncbi.nlm.nih.gov/protein/XP_031920077> <br>     whole document | 1-23 |
| A | KIM, Seung-Su et al. Engineering of dye-mediated dehydrogenase property of fructosyl amino acid oxidase by site-directed mutagenesis studies of its putative proton relay system. Biotechnol. Lett. 2010, vol. 32, pp. 1123-1129 <br>     fig. 3, table 1 | 1-23 |

Form PCT/ISA/210 (second sheet) (July 2022)

| **INTERNATIONAL SEARCH REPORT** | International application No. |
|---|---|
| | **PCT/JP2024/025602** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

  a. ☑ forming part of the international application as filed.

  b. ☐ furnished subsequent to the international filing date for the purposes of international search (Rule 13*ter*.1(a)),

  ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/025602**

| Patent document cited in search report | | | Publication date (day/month/year) | Patent family member(s) | | | Publication date (day/month/year) |
|---|---|---|---|---|---|---|---|
| JP | 2013-500729 | A | 10 January 2013 | US | 2012/0208226 | A1 | |
| | | | | fig. 1, paragraphs [0056]-[0067] | | | |
| | | | | WO | 2011/015326 | A2 | |
| | | | | EP | 2287295 | A1 | |
| | | | | CN | 102471763 | A | |
| WO | 2015/060431 | A1 | 30 April 2015 | US | 2016/0274129 | A1 | |
| | | | | fig. 1-1 to 1-5, paragraphs [0040]-[0041] | | | |
| | | | | EP | 3061829 | A1 | |
| | | | | KR | 10-2016-0068966 | A | |
| | | | | CN | 105683390 | A | |
| WO | 2019/221264 | A1 | 21 November 2019 | US | 2021/0223200 | A1 | |
| | | | | claims 1, 38, example 3, paragraph [0226] | | | |
| | | | | EP | 3812450 | A1 | |
| | | | | CN | 112292446 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019221264 A
- WO 2019045052 A
- WO 2016159384 A
- WO 2016072520 A
- WO 2015020200 A
- WO 2015096621 A
- JP 2015177790 A
- WO 2016063984 A
- JP 2009000084 A